# EUROPEAN PATENT APPLICATION

(11) **EP 3 207 930 A1**
(43) Date of publication of application: **23.08.2017**
(21) Application number: 16203755.0
(22) Date of filing: 12.01.2012
(51) Int. Cl.: A61K 31/55, A61P 37/00, C07D 223/16, C07D 243/14, C07D 401/12, C07D 487/04

(54) **SUBSTITUTED BENZOAZEPINES AS TOLL-LIKE RECEPTOR MODULATORS**

(30) Priority: 12.01.2011 US 201161432070 P
(62) Divisional of application: 12733896.0
(71) Applicant: VentiRx Pharmaceuticals, Inc., Seattle, WA 98101 (US); Array Biopharma, Inc., Boulder, CO 80301 (US)
(72) Inventor: HOWBERT, James, Jeffry, Seattle, WA 98195-5065 (US); HERSHBERG, Robert, Seattle, WA 98115 (US); BURGESS, Laurence, E., Boulder, CO 80301 (US); DOHERTY, George, A., Libertyville, IL 60048 (US); EARY, Charles Todd, Boulder, CO 80304 (US); GRONEBERG, Robert, D., Boulder, CO 80304 (US); JONES, Zachary, Broomfield, CO 80020 (US); LYSSIKATOS, Joseph P., Piedmont, CA 94611 (US); YANG, Hong Woon, Potomac, MD 20854 (US)
(74) Representative: Rupp, Christian

(57) **Abstract**

Provided are compounds of formula (II) useful for modulation of signaling through the Toll-like receptors TLR7 and/or TLR8. The compounds have use in treating or preventing disease, including cancer, autoimmune disease, fibrotic disease, cardiovascular disease, infectious disease, inflammatory disorder, graft rejection, or graft-versus-host disease.

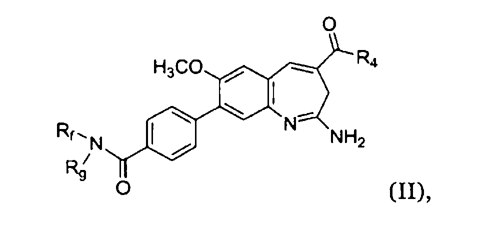

## Description

### RELATED APPLICATIONS

This application claims priority to, and the benefit of, U.S. provisional application No. 61/432,070, filed January 12, 2011, the entire content of which is incorporated herein by reference in its entirety.

### FIELD OF THE INVENTION

This invention relates to methods and compositions for modulating immune function. More specifically, this invention relates to compositions and methods for modulating TLR7- and/or TLR8-mediated signaling.

### BACKGROUND OF THE INVENTION

Stimulation of the immune system, which includes stimulation of either or both innate immunity and adaptive immunity, is a complex phenomenon that can result in either protective or adverse physiologic outcomes for the host. In recent years there has been increased interest in the mechanisms underlying innate immunity, which is believed to initiate and support adaptive immunity. This interest has been fueled in part by the recent discovery of a family of highly conserved pattern recognition receptor proteins known as Toll-like receptors (TLRs) believed to be involved in innate immunity as receptors for pathogen associated molecular patterns (PAMPs). Compositions and methods useful for modulating innate immunity are therefore of great interest, as they may affect therapeutic approaches to conditions involving autoimmunity, inflammation, allergy, asthma, graft rejection, graft versus host disease (GvHD), infection, cancer, and immunodeficiency.

Toll-like receptors (TLRs) are type I transmembrane proteins that allow organisms (including mammals) to detect microbes and initiate an innate immune response (Beutler, B., Nature 2004, 430:257-263). They contain homologous cytoplasmic domains and leucine-rich extracellular domains and typically form homodimers that sense extracellular (or internalized) signals and subsequently initiate a signal transduction cascade via adaptor molecules such as MyD88 (myeloid differentiation factor 88). There is such high homology in the cytoplasmic domains of the TLRs that, initially, it was suggested that similar signaling pathways exist for all TLRs (Re, F., Strominger, J. L., Immunobiology 2004, 209:191-198). Indeed, all TLRs can activate NF-kB and MAP kinases; however, the cytokine/chemokine release profiles derived from TLR activation appears unique to each TLR. Additionally, the signaling pathway that TLRs stimulate is very similar to the pathway that the cytokine receptor IL-1R induces. This may be due to the homology that these receptors share, i.e., TIR (Toll/IL-1R homology) domains. Once the TIR domain is activated in TLRs and MyD88 is recruited, activation of the IRAK family of serine/threonine kinases results which eventually promotes the degradation of Ik-B and activation of NF-kB (Means T. K., et al. Life Sci. 2000, 68:241¬258). While it appears that this cascade is designed to allow extracellular stimuli to promote intracellular events, there is evidence that some TLRs migrate to endosomes where signaling can also be initiated. This process may allow for intimate contact with engulfed microbes and fits with the role that these receptors play in the innate immune response (Underhill, D. M., et al., Nature 1999, 401:811-815). This process might also allow host nucleic acids, released by damaged tissues (for example, in inflammatory disease) or apoptosis to trigger a response via endosomal presentation. Among mammals, there are 11 TLRs that coordinate this rapid response. A hypothesis put forward years ago (Janeway, C. A., Jr., Cold Spring Harb. Syrup. Quant. Biol. 1989, 54:1-13) that the innate immune response initiates the adaptive immune response through the pattern of TLR activation caused by microbes has now been substantiated. Thus, the pathogen-associated molecular patterns (PAMPs) presented by a diverse group of infectious organisms results in a innate immune response involving certain cytokines, chemokines and growth factors followed by a precise adaptive immune response tailored to the infectious pathogen via antigen presentation resulting in antibody production and cytotoxic T cell generation.

Gram-negative bacterial lipopolysaccharide (LPS) has long been appreciated as an adjuvant and immune-stimulant and as a pharmacological tool for inducing an inflammatory reaction in mammals similar to septic shock. Using a genetic approach, TLR4 was identified as the receptor for LPS. The discovery that LPS is an agonist of TLR4 illustrates the usefulness of TLR modulation for vaccine and human disease therapy (Aderem, A.; Ulevitch, R. J., Nature 2000, 406:782-787). It is now appreciated that various TLR agonists can activate B cells, neutrophils, mast cells, eosinophils, endothelial cells and several types of epithelia in addition to regulating proliferation and apoptosis of certain cell types.

To date, TLR7 and TLR8, which are somewhat similar, have been characterized as receptors for single-stranded RNA found in endosomal compartments and thus thought to be important for the immune response to viral challenge. Imiquimod, an approved topical antiviral/anti-cancer drug, has recently been described as a TLR7 agonist that has demonstrated clinical efficacy in certain skin disorders (Miller R. L., et al., Int. J. Immunopharm. 1999,21:1-14). This small molecule drug has been described as a structural mimetic of ssRNA. TLR8 was first described in 2000 (Du, X., et al., European Cytokine Network 2000 (Sept.), 11(3):362-371) and was rapidly ascribed to being involved with the innate immune response to viral infection (Miettinen, M., et al., Genes and Immunity 2001 (Oct.), 2(6):349-355).

Recently it was reported that certain imidazoquinoline compounds having antiviral activity are ligands of TLR7 and TLR8 (Hemmi H., et al. (2002) Nat. Immunol. 3:196-200; Jurk M., et al. (2002) Nat. Immunol. 3:499). Imidazoquinolines are potent synthetic activators of immune cells with antiviral and antitumor properties. Using macrophages from wildtype and MyD88-deficient mice, Hemmi et al. recently reported that two imidazoquinolines, imiquimod and resiquimod (R848), induce tumor necrosis factor (TNF) and interleukin-12 (IL-12) and activate NF-icB only in wildtype cells, consistent with activation through a TLR (Hemmi H., et al. (2002) Nat. Immunol. 3:196-200). Macrophages from mice deficient in TLR7 but not other TLRs produced no detectable cytokines in response to these imidazoquinolines. In addition, the imidazoquinolines induced dose-dependent proliferation of splenic B cells and the activation of intracellular signaling cascades in cells from wildtype but not TLR7-/- mice. Luciferase analysis established that expression of human TLR7, but not TLR2 or TLR4, in human embryonic kidney cells results in NF-KB activation in response to resiquimod. The findings of Hemmi et al. thus suggest that these imidazoquinoline compounds are non-natural ligands of TLR7 that can induce signaling through TLR7. Recently it was reported that R848 is also a ligand for human TLR8 (Jurk M., et al. (2002) Nat. Immunol. 3:499).

In view of the great therapeutic potential for compounds that modulate toll-like receptors, and despite the work that has already been done, there is a substantial ongoing need to expand their use and therapeutic benefits.

### SUMMARY OF THE INVENTION

The compositions described herein are useful for modulating immune responses in vitro and in vivo. Such compositions will find use in a number of clinical applications, such as in methods for treating or preventing conditions involving unwanted immune activity, including inflammatory and autoimmune disorders.

Specifically, the invention relates to a compound having the formula I: 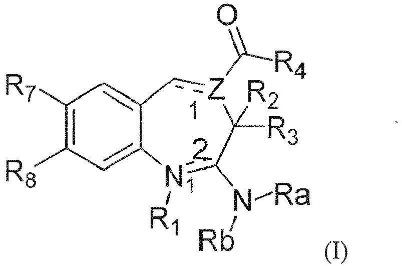 or a salt thereof, where -̅-̅-̅-̅-̅-̅ is a double bond or a single bond; R₂ and R₃ are independently selected from H and lower alkyl, or R₂ and R₃ are connected to form a saturated carbocycle having from 3 to 7 ring members; one of R₇ and R₈ is 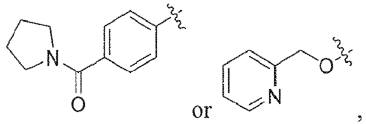 and the other is hydrogen; R₄ is -NR_{c}R_{d} or -OR₁₀; R_{c} and R_{d} are lower alkyl, where the alkyl is optionally substituted with one or more - OH; R₁₀ is alkyl, where the alkyl is optionally substituted with one or more -OH; Z is C and -̅-̅-̅-̅-̅-̅ is a double bond, or Z is N and -̅-̅-̅-̅-̅-̅ is a single bond; Rₐ and R_{b} are independently selected from H, alkyl, alkenyl, alkynyl, and Rₑ, wherein the alkyl is optionally substituted with one or more -OR₁₀, or Rₑ, Rₑ is selected from -NH₂, -NH(alkyl), and -N(alkyl)₂; R₁ is absent when -̅-̅-̅-̅-̅-̅ is a double bond, or when -̅-̅-̅-̅-̅-̅ is a single bond, N₁-R₁ and one of Rₐ or R_{b} are connected to form a saturated, partially unsaturated, or unsaturated heterocycle having 5-7 ring members and the other of Rₐ or R_{b} may be hydrogen or absent as necessary to accommodate ring unsaturation; and at least one of the following A-D applies: A) R₇ is not hydrogen B) R₈ is not hydrogen and at least one of Rₐ and R_{b} is not hydrogen; C) Z is N; or D) N₁-R₁ and one of Rₐ or R_{b} are connected to form a saturated, partially unsaturated, or unsaturated heterocycle having 5-7 ring members.

In one embodiment, R₇ of the compound of formula (I) is 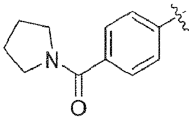 or is 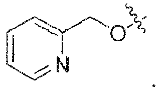 Additionally, at least one of Rₐ and R_{b} is not hydrogen in the compound of formula (I), or, for example, one of Rₐ and R_{b} is alkyl and the other of Rₐ and R_{b} is hydrogen. Further, the alkyl of formula (I) is substituted with Rₑ. In a different embodiment, both Rₐ and R_{b} are alkyl or, one of Rₐ and R_{b} is Rₑ and the other Rₐ and R_{b} is hydrogen. For example, R₈ of formula (I) is not hydrogen.

In an alternative embodiment, N₁ and one of Rₐ or R_{b} of formula (I) are connected to form a saturated, partially unsaturated, or unsaturated heterocycle having 5-7 ring members and the other of Rₐ or R_{b} is hydrogen, or absent as necessary to accommodate ring unsaturation, where the ring is a 5 membered ring, or, for example, the ring is:

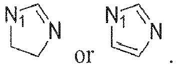

In a certain embodiment, at least one of R₂ and R₃ in the compound of formula (I) is not hydrogen, or, for example, R₂ and R₃ are connected to form a saturated carbocycle, where the saturated carbocycle is cyclopropyl. Alternatively, Z is N in the compound of formula (I).

The invention also relates to a compound having the formula II: 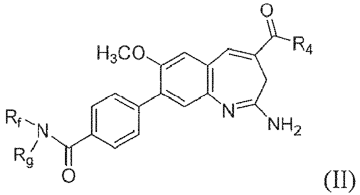 or a salt thereof, where R₄ is selected from -NR_{c}R_{d} and -OR₁₀; R_{c} and R_{d} are lower alkyl, where the alkyl is optionally substituted with one or more -OH; R₁₀ is alkyl, where the alkyl is optionally substituted with one or more -OH; R_{f} and R_{g} are lower alkyl or R_{f} and R_{g} together with the nitrogen atom to which they are attached form a saturated heterocyclic ring having 4-6 ring members. For example, R_{f} and R_{g} in the compound of formula (II), together with the nitrogen atom to which they are attached form a saturated heterocyclic ring, where the heterocyclic ring is pyrrolidine.

In an alternative embodiment, R₄ of either formula (I) or formula (II) is -OR₁₀, where R₁₀ is alkyl or is ethyl. In another embodiment, R₄ of either formula (I) or formula (II) is -NR_{c}R_{d}, where both are alkyl or both are propyl. Moreover, in certain embodiments, at least one of R_{c} or R_{d} is alkyl substituted with one -OH and at least one of R_{c} and R_{d} is 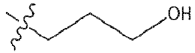 and the remaining R_{c} or R_{d} is propyl.

In some embodiments, the invention relates to a compound selected from 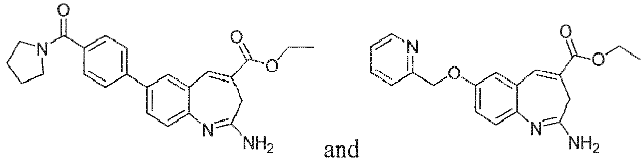 and salts thereof. Alternatively, the compound is selected from 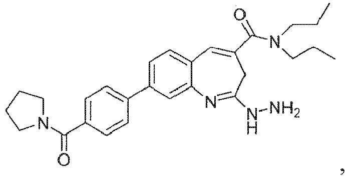 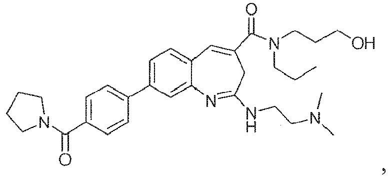 and 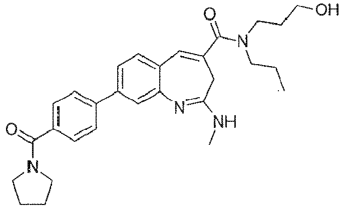 and salts thereof. In a further embodiment, the compound is either 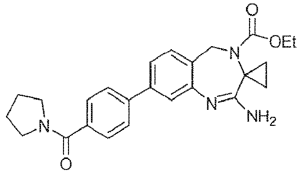 and salts thereof or 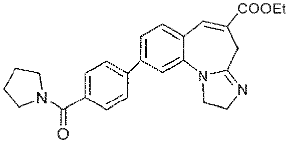 and salts thereof. Alternatively, the invention relates to a compound selected from 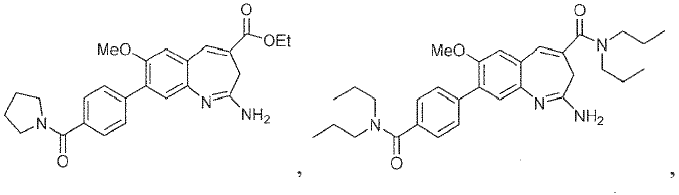 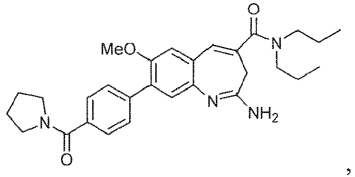 and 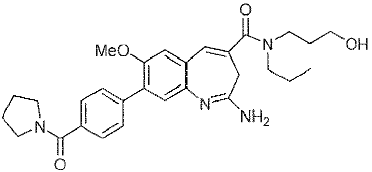 and salts thereof. In one embodiment, the compound is 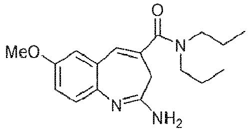 or a salt thereof. In certain embodiments, the salt of the compounds of the invention are a pharmaceutically acceptable salt. Further, the compound is a TLR8 antagonist.

Another aspect of the invention includes a kit for treating a TLR7- and/or TLR8-mediated condition that comprises a first pharmaceutical composition comprising the compounds of the invention describes *supra* and *infra;* and optionally instructions for use. Additionally, the kit includes a second pharmaceutical composition, where the second pharmaceutical composition comprises a second compound for treating a TLR7- and/or TLR8-mediated condition. The kit also comprises instructions for the simultaneous, sequential or separate administration of said first and second pharmaceutical compositions to a patient in need thereof.

The invention described herein also relates to a pharmaceutical composition, which comprises a compound or salt thereof as described *supra* and *infra* together with a pharmaceutically acceptable diluent or carrier. Additionally, the compound of the invention is used as a medicament for treating a TLR7 and/or TLR8-mediated condition in a human or animal, where the method of treating a TLR7- and/or TLR8-mediated condition includes administering to a patient, in need thereof, an effective amount of a compound described herein. Moreover, in certain embodiments, the compound is used in the manufacture of a medicament for the treatment of an autoimmune condition in a human or animal. In an alternative embodiment, the invention relates to a method of modulating a patient's immune system that includes administering to a patient in need thereof an effective amount of a compound *supra* and *infra.*

For example, a compound of the invention is a TLR8 antagonist. A TLR8 antagonist is characterized by the ability to inhibit the activation of a TLR8 receptor with an IC₅₀ of 25 µM or less. For example, a TLR8 antagonist inhibits the activation of a TLR8 receptor with an IC₅₀ of about 25 µM, 15 µM, 10 µM, 7.5 µM, 5 µM, 2.5 µM, 1.5 µM, 1 µM, 0.5 µM, 0.25 µM, 0.1 µM, 0.01 µM, or about 0.001 µM.

For example, a compound of the invention is a TLR7 antagonist. A TLR7 antagonist is characterized by the ability to inhibit the activation of a TLR7 receptor with an IC₅₀ of 25 µM or less. For example, a TLR7 antagonist inhibits the activation of a TLR7 receptor with an IC₅₀ of about 25 µM, 15 µM, 10 µM, 7.5 µM, 5 µM, 2.5 µM, 1.5 µM, 1 µM, 0.5 µM, 0.25 µM, 0.1 µM, 0.01 µM, or about 0.001 µM.

For example, a compound of the invention is a TLR7/8 antagonist. A TLR7/8 antagonist is characterized by the ability to inhibit, independently, the activation of both TLR7 and TLR8 receptors with an IC₅₀ of 25 µM or less. For example, a TLR7/8 antagonist inhibits the activation of both TLR7 and TLR8 receptors, independently, with an IC₅₀ of about 25 µM, 15 µM, 10 µM, 7.5 µM, 5 µM, 2.5 µM, 1.5 µM, 1 µM, 0.5 µM, 0.25 µM, 0.1 µM, 0.01 µM, or about 0.001 µM.

The compounds of the invention may be used in combination with other known therapeutic agents. Accordingly, this invention also relates to pharmaceutical compositions comprising a therapeutically effective amount of a compound of the invention or a salt thereof, in combination with a second therapeutic agent.

This invention further provides methods of modulating TLR7- and/or TLR8-mediated signaling, comprising contacting a cell expressing TLR7 and/or TLR8 with an effective amount of a compound of the invention, or a salt thereof. In one aspect, the method inhibits TLR7- and/or TLR8-mediated immunostimulatory signaling.

This invention further provides methods of modulating TLR7- and/or TLR8-mediated immunostimulation in a subject, comprising administering to a patient having or at risk of developing TLR7- and/or TLR8-mediated immunostimulation a compound of the invention, or a salt thereof, in an amount effective to inhibit TLR7- and/or TLR8-mediated immunostimulation in the subject.

This invention further provides methods of treating or preventing a disease or condition by modulation of TLR7- and/or TLR8-mediated cellular activities, comprising administering to a warm-blooded animal, such as a mammal, for example a human, having or at risk of developing said disease or condition, a compound of the invention, or a salt thereof.

This invention further provides methods of modulating the immune system of a mammal, comprising administering to a mammal a compound of the invention, or a salt thereof, in an amount effective to modulate said immune system.

Further provided is a compound of the invention, or a salt thereof for use as a medicament in the treatment of the diseases or conditions described herein in a mammal, for example, a human, suffering from such disease or condition. Also provided is the use of a compound of the invention, a salt thereof, in the preparation of a medicament for the treatment of the diseases and conditions described herein in a mammal, for example a human, suffering from such disease or condition.

Further provided is a compound of the invention, or a salt thereof for use as a medicament in the prevention of the diseases or conditions described herein in a mammal, for example, a human, exposed to or predisposed to the disease or condition, but the mammal does not yet experience or display symptoms of such disease or condition. Also provided is the use of a compound of the invention, a salt thereof, in the preparation of a medicament for the treatment of the diseases and conditions described herein in a mammal, for example a human, suffering from such disease or condition.

The disease or condition is selected from cancer, autoimmune disease, infectious disease, inflammatory disorder, graft rejection, and graft-versus-host disease.

This invention further provides kits comprising one or more compounds of the invention, or a salt thereof. The kit may further comprise a second compound or formulation comprising a second pharmaceutical agent.

Additional advantages and novel features of this invention shall be set forth in part in the description that follows, and in part will become apparent to those skilled in the art upon examination of the following specification or may be learned by the practice of the invention. The advantages of the invention may be realized and attained by means of the instrumentalities, combinations, compositions, and methods particularly pointed out in the appended claims.

### BRIEF DESCRIPTION OF DRAWINGS

Figure 1 is a graph depicting the dose-dependent inhibition of IL-8 production in human PBMC stimulated with CL075 following administration of certain compounds described herein.
Figure 2 is eleven graphs depicting the dose-dependent inhibition of IL-8 production in human PBMC stimulated with CL075 following administration of certain compounds described herein.

### DETAILED DESCRIPTION OF THE INVENTION

In certain aspects, the invention provides compositions and methods useful for modulating TLR7- and/or TLR8-mediated signaling. More specifically, one aspect of this invention provides a compound having the formula I: 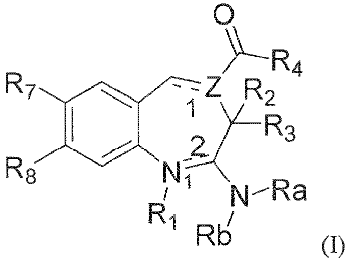 or a salt thereof, where -̅-̅-̅-̅-̅-̅ is a double bond or a single bond; R₂ and R₃ are independently selected from H and lower alkyl, or R₂ and R₃ are connected to form a saturated carbocycle having from 3 to 7 ring members; one of R₇ and R₈ is 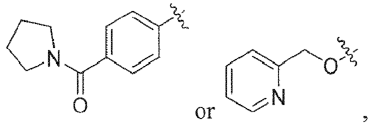 and the other is hydrogen; R₄ is -NR_{c}R_{d} or -OR₁₀; R_{c} and R_{d} are lower alkyl, where the alkyl is optionally substituted with one or more - OH; R₁₀ is alkyl, where the alkyl is optionally substituted with one or more -OH; Z is C and -̅-̅-̅-̅-̅-̅ is a double bond, or Z is N and -̅-̅-̅-̅-̅-̅ is a single bond; Rₐ and R_{b} are independently selected from H, alkyl, alkenyl, alkynyl, and Rₑ, wherein the alkyl is optionally substituted with one or more -OR₁₀, or Rₑ, Rₑ is selected from -NH₂, -NH(alkyl), and -N(alkyl)₂; R₁ is absent 2 2 when -̅-̅-̅-̅-̅-̅ is a double bond, or when -̅-̅-̅-̅-̅-̅ is a single bond, N₁-R₁ and one of Rₐ or R_{b} are connected to form a saturated, partially unsaturated, or unsaturated heterocycle having 5-7 ring members and the other of Rₐ or R_{b} may be hydrogen or absent as necessary to accommodate ring unsaturation; and at least one of the following A-D applies: A) R₇ is not hydrogen; B) R₈ is not hydrogen and at least one of Rₐ and R_{b} is not hydrogen; C) Z is N; or D) N₁-R₁ and one of Rₐ or R_{b} are connected to form a saturated, partially unsaturated, or unsaturated heterocycle having 5-7 ring members.

In one embodiment, R₇ of the compound of formula (I) is 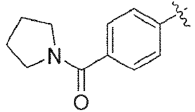 or is 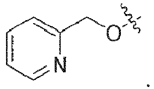 Additionally, at least one of Rₐ and R_{b} is not hydrogen in the compound of formula (I), or, for example, one of Rₐ and R_{b} is alkyl and the other of Rₐ and R_{b} is hydrogen. Further, the alkyl of formula (I) is substituted with Rₑ. In a different embodiment, both Rₐ and R_{b} are alkyl or, one of Rₐ and R_{b} is Rₑ and the other Rₐ and R_{b} is hydrogen. For example, R₈ of formula (I) is not hydrogen.

In an alternative embodiment, N₁ and one of Rₐ or R_{b} of formula (I) are connected to form a saturated, partially unsaturated, or unsaturated heterocycle having 5-7 ring members and the other of Rₐ or R_{b} is hydrogen, or absent as necessary to accommodate ring unsaturation, where the ring is a 5 membered ring, or, for example, the ring is:

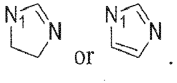

In a certain embodiment, at least one of R₂ and R₃ in the compound of formula (I) is not hydrogen, or, for example, R₂ and R₃ are connected to form a saturated carbocycle, where the saturated carbocycle is cyclopropyl. Alternatively, Z is N in the compound of formula (I).

The invention also relates to a compound having the formula II: 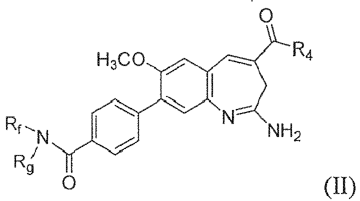 or a salt thereof, where R₄ is selected from -NR_{c}R_{d} and -OR₁₀; R_{c} and R_{d} are lower alkyl, where the alkyl is optionally substituted with one or more -OH; R₁₀ is alkyl, where the alkyl is optionally substituted with one or more -OH; R_{f} and R_{g} are lower alkyl or R_{f} and R_{g} together with the nitrogen atom to which they are attached form a saturated heterocyclic ring having 4-6 ring members. For example, R_{f} and R_{g} in the compound of formula (II), together with the nitrogen atom to which they are attached form a saturated heterocyclic ring, where the heterocyclic ring is pyrrolidine.

In an alternative embodiment, R₄ of either formula (I) or formula (II) is -OR₁₀, where R₁₀ is alkyl or is ethyl. In another embodiment, R₄ of either formula (I) or formula (II) is -NR_{c}R_{d}, where both are alkyl or both are propyl. Moreover, in certain embodiments, at least one of R_{c} or R_{d} is alkyl substituted with one -OH and at least one of R_{c} and R_{d} is 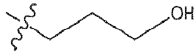 and the remaining R_{c} or R_{d} is propyl.

For example, the invention relates to a compound selected from 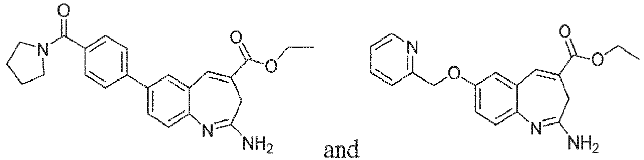 and salts thereof. Alternatively, the compound is selected from 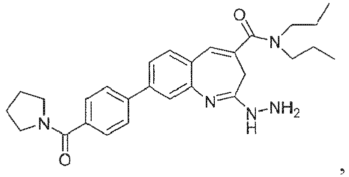 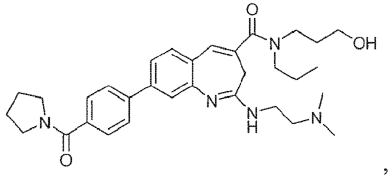 and 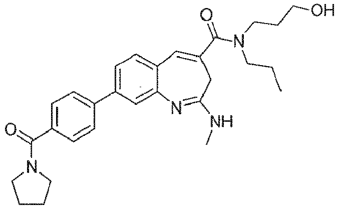 and salts thereof. In a further embodiment, the compound is either 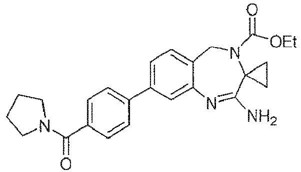 and salts thereof or 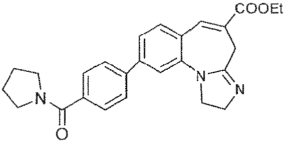 and salts thereof. Alternatively, the invention relates to a compound selected from 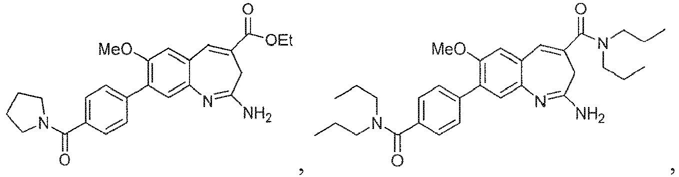 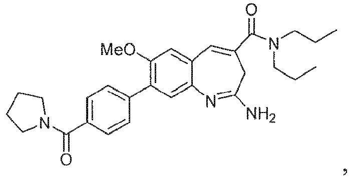 and 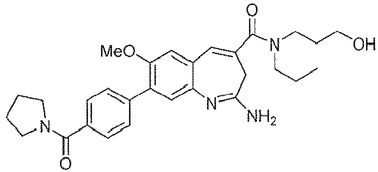 and salts thereof. For example, the compound is 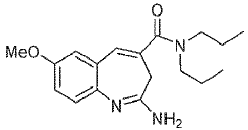 or a salt thereof. In certain embodiments, the salt of the compounds of the invention are a pharmaceutically acceptable salt. Further, the compound is a TLR8 antagonist.

Another aspect of the invention includes a kit for treating a TLR7- and/or TLR8-mediated condition that comprises a first pharmaceutical composition comprising the compounds of the invention describes *supra* and *infra;* and optionally instructions for use. Additionally, the kit includes a second pharmaceutical composition, where the second pharmaceutical composition comprises a second compound for treating a TLR7- and/or TLR8-mediated condition. The kit also comprises instructions for the simultaneous, sequential or separate administration of said first and second pharmaceutical compositions to a patient in need thereof.

The invention described herein also relates to a pharmaceutical composition, which comprises a compound or salt thereof as described *supra* and *infra* together with a pharmaceutically acceptable diluent or carrier. Additionally, the compound of the invention is used as a medicament for treating a TLR7 and/or TLR8-mediated condition in a human or animal, where the method of treating a TLR7- and/or TLR8-mediated condition includes administering to a patient, in need thereof, an effective amount of a compound described herein. Moreover, in certain embodiments, the compound is used in the manufacture of a medicament for the treatment of an autoimmune condition in a human or animal. In an alternative embodiment, the invention relates to a method of modulating a patient's immune system that includes administering to a patient in need thereof an effective amount of a compound *supra* and *infra.*

One aspect of the invention relates to a salt of a compound of the invention, wherein the salt is a pharmaceutically acceptable salt.

For example, a compound of the invention is a TLR8 antagonist. A TLR8 antagonist is characterized by the ability to inhibit the activation of a TLR8 receptor with an IC₅₀ of 25 µM or less. For example, a TLR8 antagonist inhibits the activation of a TLR8 receptor with an IC₅₀ of about 25 µM, 15 µM, 10 µM, 7.5 µM, 5 µM, 2.5 µM, 1.5 µM, 1 µM, 0.5 µM, 0.25 µM, 0.1 µM, 0.01 µM, or about 0.001 µM.

For example, a compound of the invention is a TLR7 antagonist. A TLR7 antagonist is characterized by the ability to inhibit the activation of a TLR7 receptor with an IC₅₀ of 25 µM or less. For example, a TLR7 antagonist inhibits the activation of a TLR7 receptor with an IC₅₀ of about 25 µM, 15 µM, 10 µM, 7.5 µM, 5 µM, 2.5 µM, 1.5 µM, 1 µM, 0.5 µM, 0.25 µM, 0.1 µM, 0.01 µM, or about 0.001 µM.

For example, a compound of the invention is a TLR7/8 antagonist. A TLR7/8 antagonist is characterized by the ability to inhibit, independently, the activation of both TLR7 and TLR8 receptors with an IC₅₀ of 25 µM or less. For example, a TLR7/8antagonist inhibits the activation of both TLR7 and TLR8 receptors, independently, with an IC₅₀ of about 25 µM, 15 µM, 10 µM, 7.5 µM, 5 µM, 2.5 µM, 1.5 µM, 1 µM, 0.5 µM, 0.25 µM, 0.1 µM, 0.01 µM, or about 0.001 µM.

One aspect of the invention relates to a kit for treating a TLR7- and/or TLR8-mediated condition, comprising:
a) a first pharmaceutical composition comprising a compound of the invention or salt thereof; and
b) optionally instructions for use.

In one embodiment, the invention relates to the kit further comprising (c) a second pharmaceutical composition, wherein the second pharmaceutical composition comprises a second compound for treating a TLR7- and/or TLR8-mediated condition. In one embodiment, the invention relates to the kit, further comprising instructions for the simultaneous, sequential or separate administration of said first and second pharmaceutical compositions to a patient in need thereof.

One aspect of the invention relates to a pharmaceutical composition, which comprises a compound of the invention or salt thereof, together with a pharmaceutically acceptable diluent or carrier.

One aspect of the invention relates to a compound of the invention for use as a medicament for treating a TLR7 and/or TLR8-mediated condition in a human or animal. In one embodiment, the invention relates to a compound of the invention or salt thereof, in the manufacture of a medicament for the treatment of an abnormal cell growth condition in a human or animal.

One aspect of the invention relates to a method of treating a TLR7- and/or TLR8-mediated condition, comprising administering to a patient in need thereof an effective amount of a compound of the invention or salt thereof.

One aspect of the invention relates to a method of modulating a patient's immune system, comprising administering to a patient in need thereof an effective amount of a compound of the invention or salt thereof.

The invention includes a compound selected from the compounds listed in Table 1.

**Table 1.**

| Compound No. | Structure |
|---|---|
| 63 | 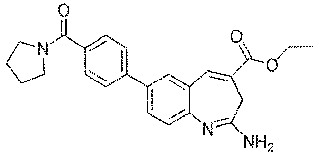 |
| 33 | 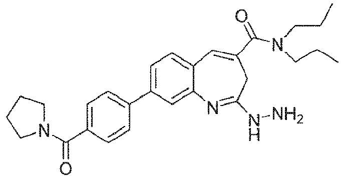 |
| 76 | 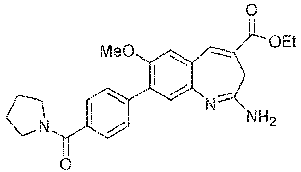 |
| 12 | 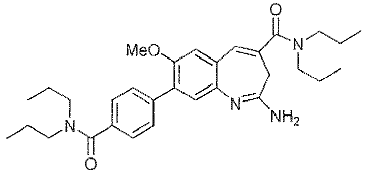 |
| 65 | 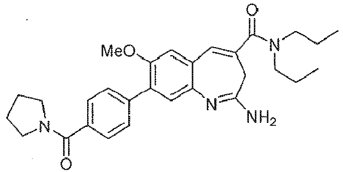 |
| 74 | 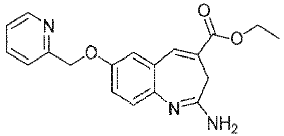 |
| 88 | 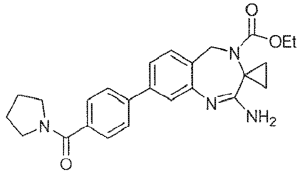 |
| 47 | 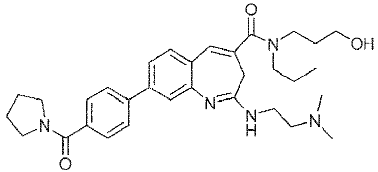 |
| 90 | 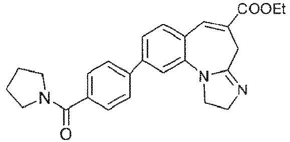 |
| 46 | 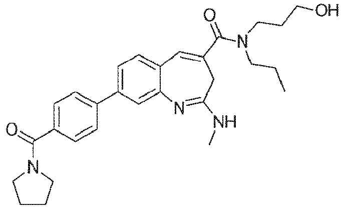 |
| 70 | 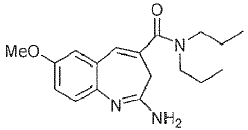 |
| 25 | 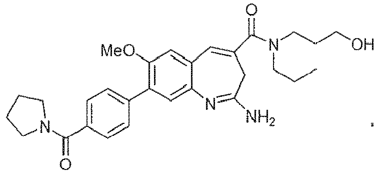 |
| 3173 | 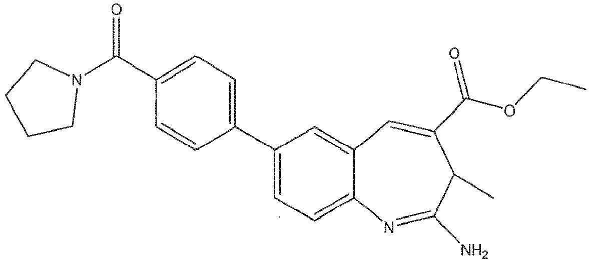 |
| 3348 | 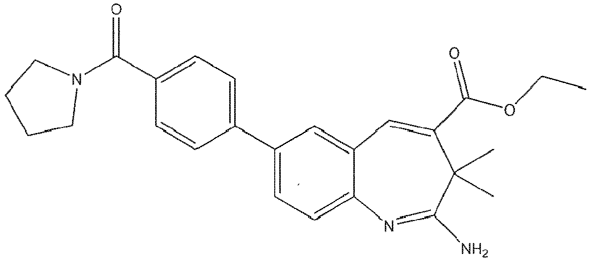 |
| 3260 | 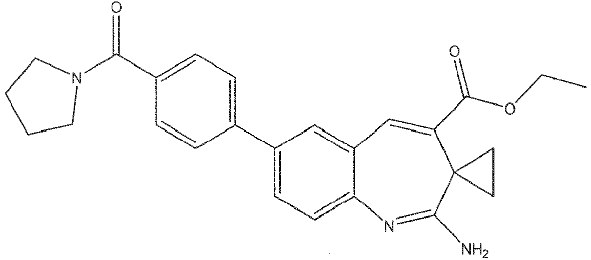 |
| 2931 | 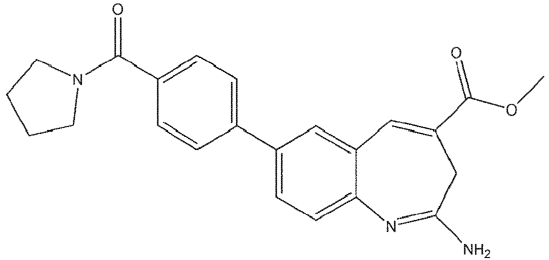 |
| 2984 | 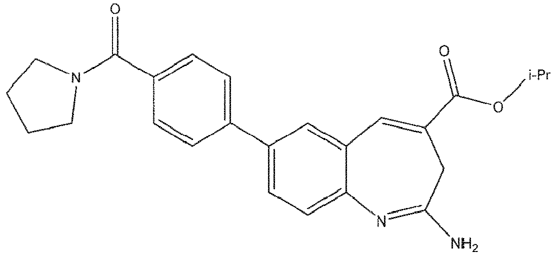 |
| 2986 | 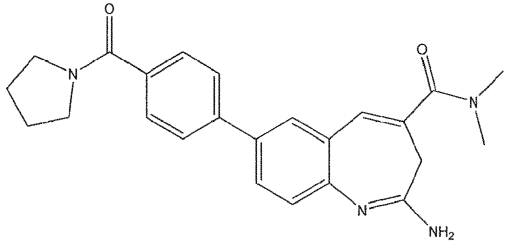 |
| 2987 | 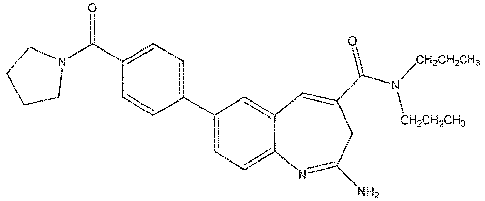 |
| 2966 | 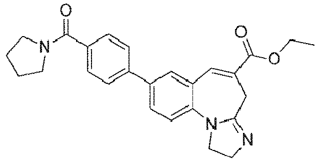 |
| 2919 | 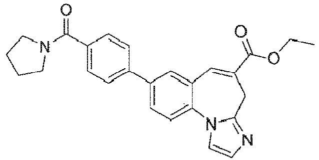 |
| 2976 | 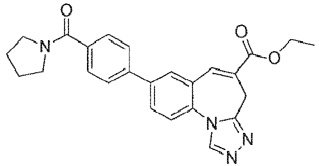 |
| 3000 | 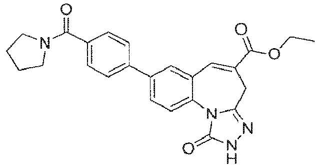 |
| 2922 | 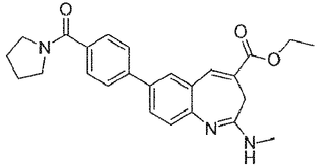 |
| 2929 | 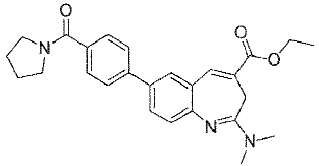 |
| 2962 | 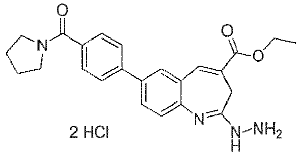 |
| 2926 | 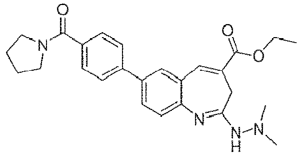 |
| 2954 | 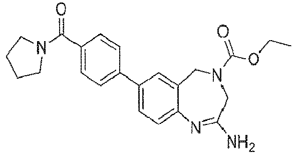 |
| 3020 | 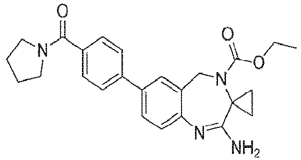 |

In one aspect, the invention includes a compound, or salt thereof, with an IC₅₀ value ≤ 25 µM for TLR8. In another aspect, the invention includes a compound or salt thereof, with an IC₅₀ value ≤15 µM for TLR8. In another aspect, the invention includes a compound or salt thereof, with an IC₅₀ value ≤10 µM for TLR8. In another aspect, the invention includes a compound or salt thereof, with an IC₅₀ value <7.5 µM for TLR8. In another aspect, the invention includes a compound or salt thereof, with an IC₅₀ value ≤5 µM for TLR8. In another aspect, the invention includes a compound or salt thereof, with an IC₅₀ value ≤2.5 µM for TLR8. In another aspect, the invention includes a compound or salt thereof, with an IC₅₀ value ≤1.5 µM for TLR8. In another aspect, the invention includes a compound or salt thereof, with an IC₅₀ value ≤1 µM for TLR8. In another aspect, the invention includes a compound or salt thereof, with an IC₅₀ value ≤0.5 µM for TLR8. In another aspect, the invention includes a compound or salt thereof, with an IC₅₀ value ≤0.25 µM for TLR8. In another aspect, the invention includes a compound or salt thereof, with an IC₅₀ value ≤0.1 µM for TLR8. In another aspect, the invention includes a compound or salt thereof, with an IC₅₀ value ≤0.01 µM for TLR8. In another aspect, the invention includes a compound or salt thereof, with an IC₅₀ value ≤0.001 µM forTLR8.

In one aspect, the invention includes a compound, or salt thereof, with an IC₅₀ value ≤ 25 µM for TLR7. In another aspect, the invention includes a compound or salt thereof, with an IC₅₀ value ≤15 µM for TLR7. In another aspect, the invention includes a compound or salt thereof, with an IC₅₀ value ≤10 µM for TLR7. In another aspect, the invention includes a compound or salt thereof, with an IC₅₀ value ≤7.5 µM for TLR7 In another aspect, the invention includes a compound or salt thereof, with an IC₅₀ value ≤5 µM for TLR7. In another aspect, the invention includes a compound or salt thereof, with an IC₅₀ value ≤2.5 µM for TLR7. In another aspect, the invention includes a compound or salt thereof, with an IC₅₀ value ≤1.5 µM for TLR7. In another aspect, the invention includes a compound or salt thereof, with an IC₅₀ value ≤1 µM for TLR7. In another aspect, the invention includes a compound or salt thereof, with an IC₅₀ value ≤0.5 µM for TLR7. In another aspect, the invention includes a compound or salt thereof, with an IC₅₀ value ≤0.25 µM for TLR7. In another aspect, the invention includes a compound or salt thereof, with an IC₅₀ value ≤0.1 µM for TLR7. In another aspect, the invention includes a compound or salt thereof, with an IC₅₀ value ≤0.01 µM for TLR7. In another aspect, the invention includes a compound or salt thereof, with an IC₅₀ value ≤0.001 µM for TLR7.

In one aspect, the invention does not include a compound or salt thereof, with an IC₅₀ > 25 µM for TLR7. In one aspect, the invention does not include a compound or salt thereof, with an IC₅₀ > 25 µM for TLR8. In one aspect, the invention does not include a compound or salt thereof, with an IC₅₀ value > 25 µM for TLR7 and for TLR8.

In one embodiment, the TLR7, TLR8, or TLR7/8 antagonist activity of a compound of the invention is measured relative to the activity of a known TLR7, TLR8, or TLR7/8 agonist. See, for example, compounds described in PCT publication WO 2007/024612.

The term "compound of the invention" refers to exemplified compounds and compounds covered under the formulae described herein.

The term "substituted," as used herein, means that any one or more hydrogen atoms on the designated atom is replaced with a selection from the indicated group, provided that the designated atom's normal valency is not exceeded, and that the substitution results in a stable compound. When a substituent is keto (i.e., =O), then 2 hydrogens on the atom are replaced. Ring double bonds, as used herein, are double bonds that are formed between two adjacent ring atoms (e.g., C=C, C=N, or N=N).

A chemical structure showing a dashed line representation for a chemical bond indicates that the bond is optionally present. For example, a dashed line drawn next to a solid single bond indicates that the bond can be either a single bond or a double bond.

The term "alkyl" as used herein refers to a saturated linear or branched-chain monovalent hydrocarbon radical having one to twelve, including one to ten carbon atoms (C₁-C₁₀), one to six carbon atoms (C₁-C₆) and one to four carbon atoms (C₁-C₄), wherein the alkyl radical may be optionally substituted independently with one or more substituents described below. Lower alkyl means an alkyl group having one to six carbon atoms (C₁-C₆). Examples of alkyl radicals include hydrocarbon moieties such as, but not limited to: methyl (Me, -CH₃), ethyl (Et, -CH₂CH₃), 1-propyl (n-Pr, n-propyl, -CH₂CH₂CH₃), 2-propyl (i-Pr, i-propyl, - CH(CH₃)₂), 1-butyl (n-Bu, n-butyl, -CH₂CH₂CH₂CH₃), 2-methyl- 1-propyl (i-Bu, i-butyl, - CH₂CH(CH₃)₂), 2-butyl (s-Bu, s-butyl, -CH(CH₃)CH₂CH₃), 2-methyl-2-propyl (t-Bu, t-butyl, - C(CH₃)₃), 1-pentyl (n-pentyl, -CH₂CH₂CH₂CH₂CH₃), 2-pentyl (-CH(CH₃)CH₂CH₂CH₃), 3-pentyl (-CH(CH₂CH₃)₂), 2-methyl-2-butyl (-C(CH₃)₂CH₂CH₃), 3-methyl-2-butyl (-CH(CH₃)CH(CH₃)₂), 3-methyl-1-butyl (-CH₂CH₂CH(CH₃)₂), 2-methyl- 1 -butyl (-CH₂CH(CH₃)CH₂CH₃), 1-hexyl (-CH₂CH₂CH₂CH₂CH₂CH₃), 2-hexyl (-CH(CH₃)CH₂CH₂CH₂CH₃), 3-hexyl (-CH(CH₂CH₃)(CH₂CH₂CH₃)), 2-methyl-2-pentyl (-C(CH₃)₂CH₂CH₂CH₃), 3-methyl-2-pentyl (-CH(CH₃)CH(CH₃)CH₂CH₃), 4-methyl-2-pentyl (-CH(CH₃)CH₂CH(CH₃)₂), 3-methyl-3-pentyl (-C(CH₃)(CH₂CH₃)₂), 2-methyl-3-pentyl (-CH(CH₂CH₃)CH(CH₃)₂), 2,3-dimethyl-2-butyl (-C(CH₃)₂CH(CH₃)₂), 3,3-dimethyl-2-butyl (-CH(CH₃)C(CH₃)₃, 1-heptyl, and 1-octyl.

Moieties replacing a hydrogen atom on a "substituted" radical include, for example, halogen, lower alkyl, lower alkoxy, keto, amino, alkylamino, dialkylamino, trifluoromethyl, aryl, heteroaryl and hydroxyl.

The term "alkenyl" refers to a linear or branched-chain monovalent hydrocarbon radical having two to 10 carbon atoms (C₂-C₁₀), including two to six carbon atoms (C₂-C₆) and two to four carbon atoms (C₂-C₄), and at least one double bond, and includes, but is not limited to, ethenyl, propenyl, 1-but-3-enyl, 1-pent-3-enyl, 1-hex-5-enyl and the like, wherein the alkenyl radical may be optionally substituted independently with one or more substituents described herein, and includes radicals having "cis" and "trans" orientations, or alternatively, "E" and "Z" orientations. The term "alkenyl" includes allyl.

The term "alkynyl" refers to a linear or branched monovalent hydrocarbon radical of two to twelve carbon atoms (C₂-C₁₂), including two to 10 carbon atoms (C₂-C₁₀), two to six carbon atoms (C₂-C₆) and two to four carbon atoms (C₂-C₄), containing at least one triple bond. Examples include, but are not limited to, ethynyl, propynyl, butynyl, pentyn-2-yl and the like, wherein the alkynyl radical may be optionally substituted independently with one or more substituents described herein.

The terms "carbocycle," "carbocyclyl," or "cycloalkyl" are used interchangeably herein and refer to saturated or partially unsaturated cyclic hydrocarbon radical having from three to twelve carbon atoms (C₃-C₁₂), including from three to ten carbon atoms (C₃-C₁₀) and from three to six carbon atoms (C₃-C₆). The term "cycloalkyl" includes monocyclic and polycyclic (e.g., bicyclic and tricyclic) cycloalkyl structures, wherein the polycyclic structures optionally include a saturated or partially unsaturated cycloalkyl fused to a saturated or partially unsaturated cycloalkyl or heterocycloalkyl ring or an aryl or heteroaryl ring. Examples of cycloalkyl groups include, but are not limited to, cyclopropyl, cyclobutyl, cyclopentyl, cyclohexyl, cycloheptyl, and the like. Bicyclic carbocycles have 7 to 12 ring atoms, e.g. arranged as a bicyclo [4,5], [5,5], [5,6] or [6,6] system, or 9 or 10 ring atoms arranged as a bicyclo [5,6] or [6,6] system, or as bridged systems such as bicyclo[2.2.1]heptane, bicyclo[2.2.2]octane, and bicyclo[3.2.2]nonane. The cycloalkyl may be optionally substituted independently at one or more substitutable positions with one or more substituents described herein. Such cycloalkyl groups may be optionally substituted with, for example, one or more groups independently selected from C₁-C₆ alkyl, C₁-C₆ alkoxy, halogen, hydroxy, cyano, nitro, amino, mono(C₁ C₆)alkylamino, di(C₁-C₆)alkylamino, C₂-C₆ alkenyl, C₂-C₆ alkynyl, C₁-C₆ haloalkyl, C₁-C₆ haloalkoxy, amino(C₁-C₆)alkyl, mono (C₁-C₆)alkylamino (C₁-C₆)alkyl and di(C₁ C₆)alkylamino(C₁-C₆)alkyl.

The terms "heterocycloalkyl," "heterocycle" and "heterocyclyl" are used interchangeably herein and refer to a saturated or partially unsaturated carbocyclic radical of 3 to 8 ring atoms in which at least one ring atom is a heteroatom selected from nitrogen, oxygen and sulfur, the remaining ring atoms being C, where one or more ring atoms may be optionally substituted independently with one or more substituents described below. The radical may be a carbon radical or heteroatom radical. The term "heterocycle" includes heterocycloalkoxy. The term further includes fused ring systems which include a heterocycle fused to an aromatic group. "Heterocycloalkyl" also includes radicals where heterocycle radicals are fused with aromatic or heteroaromatic rings. Examples of heterocycloalkyl rings include, but are not limited to, pyrrolidinyl, tetrahydrofuranyl, dihydrofuranyl, tetrahydrothienyl, tetrahydropyranyl, dihydropyranyl, tetrahydrothiopyranyl, piperidino, morpholino, thiomorpholino, thioxanyl, piperazinyl, homopiperazinyl, azetidinyl, oxetanyl, thietanyl, homopiperidinyl, oxepanyl, thiepanyl, oxazepinyl, diazepinyl, thiazepinyl, 1,2,3,6- tetrahydropyridinyl, 2-pyrrolinyl, 3-pyrrolinyl, indolinyl, 2H-pyranyl, 4H-pyranyl, dioxanyl, 1,3-dioxolanyl, pyrazolinyl, dithianyl, dithiolanyl, dihydropyranyl, dihydrothienyl, dihydrofuranyl, pyrazolidinylimidazolinyl, imidazolidinyl, 3-azabicyco[3.1.0]hexanyl, 3-azabicyclo[4.1.0]heptanyl, azabicyclo[2.2.2]hexanyl, 3H-indolyl quinolizinyl and N-pyridyl ureas. Spiro moieties are also included within the scope of this definition. The foregoing groups, as derived from the groups listed above, may be C-attached or N-attached where such is possible. For instance, a group derived from pyrrole may be pyrrol-1-yl (N-attached) or pyrrol-3-yl (C-attached). Further, a group derived from imidazole may be imidazol-1-yl (N attached) or imidazol-3-yl (C-attached). An example of a heterocyclic group wherein 2 ring carbon atoms are substituted with oxo (=O) moieties is 1,1-dioxo-thiomorpholinyl. The heterocycle groups herein are unsubstituted or, as specified, substituted in one or more substitutable positions with various groups. For example, such heterocycle groups may be optionally substituted with, for example, one or more groups independently selected from C₁-C₆ alkyl, C₁-C₆ alkoxy, halogen, hydroxy, cyano, nitro, amino, mono(C₁-C₆)alkylamino, di(C₁-C₆6)alkylamino, C₂-C₆ alkenyl, C₂-C₆ alkynyl, C₁-C₆ haloalkyl, C₁-C₆ haloalkoxy, amino (C₁-C₆)alkyl, mono (C₁-C₆)alkylamino(C₁-C₆)alkyl or di(C₁-C₆)alkylamino(C₁-C₆)alkyl.

The term "aryl" refers to a monovalent aromatic carbocyclic radical having a single ring (e.g., phenyl), multiple rings (e.g., biphenyl), or multiple condensed rings in which at least one is aromatic, (e.g., 1,2,3,4-tetrahydronaphthyl, naphthyl, etc.), which is optionally substituted with one or more substituents independently selected from, for example, halogen, lower alkyl, lower alkoxy, trifluoromethyl, aryl, heteroaryl and hydroxy.

The term "heteroaryl" refers to a monovalent aromatic radical of 5-, 6-, or 7-membered rings and includes fused ring systems (at least one of which is aromatic) of 5-10 atoms containing at least one and up to four heteroatoms selected from nitrogen, oxygen, and sulfur. Examples of heteroaryl groups are pyridinyl, imidazolyl, pyrimidinyl, pyrazolyl, triazolyl, pyrazinyl, tetrazolyl, furyl, thienyl, isoxazolyl, thiazolyl, oxazolyl, isothiazolyl, pyrrolyl, quinolinyl, isoquinolinyl, indolyl, benzimidazolyl, benzofuranyl, cinnolinyl, indazolyl, indolizinyl, phthalazinyl, pyridazinyl, triazinyl, isoindolyl, pteridinyl, purinyl, oxadiazolyl, triazolyl, thiadiazolyl, thiadiazolyl, furazanyl, benzofurazanyl, benzothiophenyl, benzothiazolyl, benzoxazolyl, quinazolinyl, quinoxalinyl, naphthyridinyl, isobenzofuran-1(3H)-one, and furopyridinyl. Spiro moieties are also included within the scope of this definition. Heteroaryl groups are optionally substituted with one or more substituents independently selected from, for example, halogen, lower alkyl, lower alkoxy, haloalkyl, aryl, heteroaryl, and hydroxy.

The compounds of this invention may possess one or more asymmetric centers; such compounds can therefore be produced as individual (R)- or (S)-stereoisomers or as mixtures thereof. Unless indicated otherwise, the description or naming of a particular compound in the specification and claims is intended to include both individual enantiomers, diastereomers mixtures, racemic or otherwise, thereof. Accordingly, this invention also includes all such isomers, including diastereomeric mixtures, pure diastereomers and pure enantiomers of the compounds.

Diastereomeric mixtures can be separated into their individual diastereomers on the basis of their physical chemical differences by methods known to those skilled in the art, for example, by chromatography or fractional crystallization. Enantiomers can be separated by converting the enantiomer mixture into a diastereomeric mixture by reaction with an appropriate optically active compound (e.g., alcohol), separating the diastereomers and converting (e.g., hydrolyzing) the individual diastereomers to the corresponding pure enantiomers. Enantiomers can also be separated by use of a chiral HPLC column. Methods for the determination of stereochemistry and the separation of stereoisomers are well known in the art (see discussion in Chapter 4 of "Advanced Organic Chemistry", 4th edition, J. March, John Wiley and Sons, New York, 1992).

In the structures shown herein, where the stereochemistry of any particular chiral atom is not specified, then all stereoisomers are contemplated and included as the compounds of the invention. Where stereochemistry is specified by a solid wedge or dashed line representing a particular configuration, then that stereoisomer is so specified and defined.

A single stereoisomer, e.g. an enantiomer, substantially free of its stereoisomer may be obtained by resolution of the racemic mixture using a method such as formation of diastereomers using optically active resolving agents (Eliel, E. and Wilen, S. Stereochemistry of Organic Compounds, John Wiley & Sons, Inc., New York, 1994; Lochmuller, C. H., (1975) J. Chromatogr., 113(3):283-302). Racemic mixtures of chiral compounds of the invention can be separated and isolated by any suitable method, including: (1) formation of ionic, diastereomeric salts with chiral compounds and separation by fractional crystallization or other methods, (2) formation of diastereomeric compounds with chiral derivatizing reagents, separation of the diastereomers, and conversion to the pure stereoisomers, and (3) separation of the substantially pure or enriched stereoisomers directly under chiral conditions. See: Drug Stereochemistry, Analytical Methods and Pharmacology, Irving W. Wainer, Ed., Marcel Dekker, Inc., New York (1993).

Under method (1), diastereomeric salts can be formed by reaction of enantiomerically pure chiral bases such as brucine, quinine, ephedrine, strychnine, a-methyl-13-phenylethylamine (amphetamine), and the like with asymmetric compounds bearing acidic functionality, such as carboxylic acid and sulfonic acid. The diastereomeric salts may be induced to separate by fractional crystallization or ionic chromatography. For separation of the optical isomers of amino compounds, addition of chiral carboxylic or sulfonic acids, such as camphorsulfonic acid, tartaric acid, mandelic acid, or lactic acid can result in formation of the diastereomeric salts.

Alternatively, by method (2), the substrate to be resolved is reacted with one enantiomer of a chiral compound to form a diastereomeric pair (E. and Wilen, S."Stereochemistry of Organic Compounds", John Wiley & Sons, Inc., 1994, p. 322). Diastereomeric compounds can be formed by reacting asymmetric compounds with enantiomerically pure chiral derivatizing reagents, such as menthyl derivatives, followed by separation of the diastereomers and hydrolysis to yield the pure or enriched enantiomer. A method of determining optical purity involves making chiral esters, for example a menthyl ester such as (-) menthyl chloroformate, in the presence of base, or Mosher ester, a-methoxy-a-(trifluoromethyl)phenyl acetate (Jacob III, (1982) J. Org. Chem. 47:4165), of the racemic mixture, and analyzing the NMR spectrum for the presence of the two atropisomeric enantiomers or diastereomers. Stable diastereomers of atropisomeric compounds can be separated and isolated by normal- and reverse-phase chromatography following methods for separation of atropisomeric naphthyl-isoquinolines (WO 96/15111). By method (3), a racemic mixture of two enantiomers can be separated by chromatography using a chiral stationary phase (Chiral Liquid Chromatography (1989) W. J. Lough, Ed., Chapman and Hall, New York; Okamoto, (1990) J. of Chromatogr. 513:375-378). Enriched or purified enantiomers can be distinguished by methods used to distinguish other chiral molecules with asymmetric carbon atoms, such as optical rotation and circular dichroism.

The present invention is intended to include all isotopes of atoms occurring in the present compounds. Isotopes include those atoms having the same atomic number but different mass numbers. By way of general example and without limitation, isotopes of hydrogen include tritium and deuterium, and isotopes of carbon include C-13 and C-14.

In addition to compounds of the invention, the invention also includes pharmaceutically acceptable salts of such compounds.

A "pharmaceutically acceptable salt," unless otherwise indicated, includes salts that retain the biological effectiveness of the free acids and bases of the specified compound and that are not biologically or otherwise undesirable. A compound of the invention may possess a sufficiently acidic, a sufficiently basic, or both functional groups, and accordingly react with any of a number of inorganic or organic bases, and inorganic and organic acids, to form a pharmaceutically acceptable salt. Examples of pharmaceutically acceptable salts include those salts prepared by reaction of the compounds of the present invention with a mineral or organic acid or an inorganic base, such salts including sulfates, pyrosulfates, bisulfates, sulfites, bisulfites, phosphates, monohydrogenphosphates, dihydrogenphosphates, metaphosphates, pyrophosphates, chlorides, bromides, iodides, acetates, propionates, decanoates, caprylates, acrylates, formates, isobutyrates, caproates, heptanoates, propiolates, oxalates, malonates, succinates, suberates, sebacates, fumarates, maleates, butyn-1,4-dioates, hexyne-1,6-dioates, benzoates, chlorobenzoates, methylbenzoates, dinitrobenzoates, hydroxybenzoates, methoxybenzoates, phthalates, sulfonates, xylenesulfonates, pheylacetates, phenylpropionates, phenylbutyrates, citrates, lactates, y-hydroxybutyrates, glycollates, tartrates, methanesulfonates, propanesulfonates, naphthalene-1-sulfonates, naphthalene-2-sulfonates, and mandelates. Since a single compound of the present invention may include more than one acidic or basic moiety, the compounds of the present invention may include mono, di or tri-salts in a single compound.

If the inventive compound is a base, the desired pharmaceutically acceptable salt may be prepared by any suitable method available in the art, for example, treatment of the free base with an acidic compound, particularly an inorganic acid, such as hydrochloric acid, hydrobromic acid, sulfuric acid, nitric acid, phosphoric acid and the like, or with an organic acid, such as acetic acid, maleic acid, succinic acid, mandelic acid, fumaric acid, malonic acid, pyruvic acid, oxalic acid, glycolic acid, salicylic acid, a pyranosidyl acid such as glucuronic acid or galacturonic acid, an alpha hydroxy acid such as citric acid or tartaric acid, an amino acid such as aspartic acid or glutamic acid, an aromatic acid such as benzoic acid or cinnamic acid, a sulfonic acid such as p-toluenesulfonic acid or ethanesulfonic acid, or the like.

If the inventive compound is an acid, the desired pharmaceutically acceptable salt may be prepared by any suitable method, for example, treatment of the free acid with an inorganic or organic base. Examples of suitable inorganic salts include those formed with alkali and alkaline earth metals such as lithium, sodium, potassium, barium and calcium. Examples of suitable organic base salts include, for example, ammonium, dibenzylammonium, benzylammonium, 2-hydroxyethylammonium, bis(2-hydroxyethyl)ammonium, phenylethylbenzylamine, dibenzylethylenediamine, and the like salts. Other salts of acidic moieties may include, for example, those salts formed with procaine, quinine and N-methylglucosamine, plus salts formed with basic amino acids such as glycine, ornithine, histidine, phenylglycine, lysine and arginine.

The present invention also provides salts of compounds of the invention which are not necessarily pharmaceutically acceptable salts, but which may be useful as intermediates for preparing and/or purifying compounds of the invention and/or for separating enantiomers of compounds of the invention.

It is noted that some of the preparations of compounds of the invention described herein may require protection of remote functionalities. The need for such protection will vary depending on the nature of the functionality and the conditions used in the preparation methods and can be readily determined by those skilled in the art. Such protection/deprotection methods are well known to those skilled in the art.

The compounds of the invention find use in a variety of applications. For example, in certain aspects the invention provides methods for modulating TLR7- and/or TLR8-mediated signaling. The methods of the invention are useful, for example, when it is desirable to alter TLR7- and/or TLR8-mediated signaling in response to a suitable TLR7 and/or TLR8 ligand or a TLR7 and/or TLR8 signaling agonist.

As used herein, the terms "TLR7 and/or TLR8 ligand," "ligand for TLR7 and/or TLR8," and "TLR7 and/or TLR8 signaling agonist" refer to a molecule, other than a compound of the invention, that interacts directly or indirectly with TLR7 and/or TLR8 and induces TLR7-and/or TLR8 -mediated signaling. In certain embodiments, a TLR7 and/or TLR8 ligand is a natural ligand, i.e., a TLR7 and/or TLR8 ligand that is found in nature. In certain embodiments, a TLR7 and/or TLR8 ligand refers to a molecule other than a natural ligand of TLR7 and/or TLR8, e.g., a molecule prepared by human activity.

The term "modulate" as used herein with respect to the TLR7 and/or TLR8 receptors means the mediation of a pharmacodynamic response in a subject by (i) inhibiting the receptor, or (ii) directly or indirectly affecting the normal regulation of the receptor activity.

The term "agonist" refers to a compound that, in combination with a receptor (e.g., a TLR), can produce a cellular response. An agonist may be a ligand that directly binds to the receptor. Alternatively, an agonist may combine with a receptor indirectly by, for example, (a) forming a complex with another molecule that directly binds to the receptor, or (b) otherwise resulting in the modification of another compound so that the other compound directly binds to the receptor. An agonist may be referred to as an agonist of a particular TLR (e.g., a TLR7 and/or TLR8 agonist). The term "partial agonist" refers to a compound that produces a partial but not a full cellular response.

The term "antagonist" as used herein refers to a compound that competes with an agonist or partial agonist for binding to a receptor, thereby blocking the action of an agonist or partial agonist on the receptor. More specifically, an antagonist is a compound that inhibits the activity of a TRL7 or TLR8 agonist at the TLR7 or TLR8 receptor, respectively. "Inhibit" refers to any measurable reduction of biological activity. Thus, as used herein, "inhibit" or "inhibition" may be referred to as a percentage of a normal level of activity.

In one aspect of this invention, a method of treating or preventing a condition or disorder treatable by modulation of TLR7- and/or TLR8-mediated cellular activities in a subject comprises administering to said subject a composition comprising a compound of the invention in an amount effective to treat or prevent the condition or disorder. The term "TLR7- and/or TLR8-mediated" refers to a biological or biochemical activity that results from TLR7- and/or TLR8 function.

Conditions and disorders that can be treated by the methods of this invention include, but are not limited to, cancer, immune complex-associated diseases, autoimmune diseases or disorders, inflammatory disorders, immunodeficiency, graft rejection, graft-versus-host disease, allergies, cardiovascular disease, fibrotic disease, asthma, infection, and sepsis. More specifically, methods useful in the treatment of these conditions will employ compounds of the invention that inhibit TLR7- and/or TLR8- mediated signaling. In some instances the compositions can be used to inhibit TLR7- and/or TLR8-mediated signaling in response to a TLR7 and/or TLR8 ligand or signaling agonist. In other instances the compositions can be used to inhibit TLR7- and/or TLR8-mediated immunostimulation in a subject.

The term "treating" as used herein, unless otherwise indicated, means at least the mitigation of a disease or condition and includes, but is not limited to, modulating and/or inhibiting an existing disease or condition, and/or alleviating the disease or condition to which such term applies, or one or more symptoms of such disease or condition. The term "treatment," as used herein, unless otherwise indicated, refers to the act of treating as "treating" is defined immediately above. Therapeutic treatment refers to treatment initiated after observation of symptoms and/or a suspected exposure to a causative agent of the disease or condition. Generally, therapeutic treatment may reduce the severity and/or duration of symptoms associated with the disease or condition.

As used herein, "preventing" means causing the clinical symptoms of a disease or condition not to develop i.e., inhibiting the onset of a disease or condition in a subject that may be exposed to or predisposed to the disease or condition, but does not yet experience or display symptoms of the disease or condition. Prophylactic treatment means that a compound of the invention is administered to a subject prior to observation of symptoms and/or a suspected exposure to a causative agent of the condition (e.g., a pathogen or carcinogen). Generally, prophylactic treatment may reduce (a) the likelihood that a subject that receives the treatment develops the condition and/or (b) the duration and/or severity of symptoms in the event the subject develops the condition.

As used herein, the terms "autoimmune disease," "autoimmune disorder" and "autoimmunity" refer to immunologically mediated acute or chronic injury to a tissue or organ derived from the host. The terms encompass both cellular and antibody-mediated autoimmune phenomena, as well as organ-specific and organ-nonspecific autoimmunity. Autoimmune diseases include insulin-dependent diabetes mellitus, rheumatoid arthritis, systemic lupus erythematosus, multiple sclerosis, atherosclerosis, and inflammatory bowel disease. Autoimmune diseases also include, without limitation, ankylosing spondylitis, autoimmune hemolytic anemia, Bechet's syndrome, Goodpasture's syndrome, Graves' disease, Guillain Barre syndrome, Hashimoto's thyroiditis, idiopathic thrombocytopenia, myasthenia gravis, pernicious anemia, polyarteritis nodosa, polymyositis/dermatomyositis, primary biliary sclerosis, psoriasis, sarcoidosis, sclerosing cholangitis, Sjogren's syndrome, systemic sclerosis (scleroderma and CREST syndrome), Takayasu's arteritis, temporal arteritis, and Wegener's granulomatosis. Autoimmune diseases also include certain immune complex-associated diseases.

As used here in, the term "fibrotic disease" refers to diseases or disorders involving excessive and persistent formation of scar tissue associated with organ failure in a variety of chronic diseases affecting the lungs, kidneys, eyes, heart, liver, and skin. Although tissue remodeling and scarring is part of the normal wound healing process, repeated injury or insult can lead to persistent and excessive scarring and, ultimately, organ failure.

Fibrotic conditions include diffuse fibrotic lung disease, chronic kidney disease, including diabetic kidney disease; liver fibrosis (e.g., chronic liver disease (CLD) caused by continuous and repeated insults to the liver from causes such as are viral hepatitis B and C, alcoholic cirrhosis or non-alcoholic fatty liver disease (NAFLD), or primary sclerosing cholangitis (PSC), a rare disease characterized by fibrosing inflammatory destruction of the bile ducts inside and outside the liver, leading to bile stasis, liver fibrosis, and ultimately to cirrhosis, and end-stage liver disease); lung fibrosis (e.g., idiopathic pulmonary fibrosis (IPF)); and systemic sclerosis (a degenerative disorder in which excessive fibrosis occurs in multiple organ systems, including the skin, blood vessels, heart, lungs, and kidneys).

Other examples include cystic fibrosis of the pancreas and lungs; injection fibrosis, which can occur as a complication of intramuscular injections, especially in children; endomyocardial fibrosis; mediastinal fibrosis, myelofibrosis; retroperitoneal fibrosis; progressive massive fibrosis, a complication of coal workers' pneumoconiosis; nephrogenic systemic fibrosis; and complication of certain types of surgical implants (e.g. occurrence in attempts at creating an artificial pancreas for the treatment of diabetes mellitus.

As used herein, the term "cardiovascular disease" refers to diseases or disorders of the cardiovascular system involving an imflammatory component, and/or the accumulation of plaque, including without limitation coronary artery disease, cerebrovascular disease, peripheral arterial disease, atherosclerosis, and arteriosclerosis.

As used herein, the terms "cancer" and, "tumor" refer to a condition in which abnormally replicating cells of host origin are present in a detectable amount in a subject. The cancer can be a malignant or non-malignant cancer. Cancers or tumors include, but are not limited to, biliary tract cancer; brain cancer; breast cancer; cervical cancer; choriocarcinoma; colon cancer; endometrial cancer; esophageal cancer; gastric (stomach) cancer; intraepithelial neoplasms; leukemias; lymphomas; liver cancer; lung cancer (e.g., small cell and non-small cell); melanoma; neuroblastomas; oral cancer; ovarian cancer; pancreatic cancer; prostate cancer; rectal cancer; renal (kidney) cancer; sarcomas; skin cancer; testicular cancer; thyroid cancer; as well as other carcinomas and sarcomas. Cancers can be primary or metastatic.

As used herein, the terms "inflammatory disease" and inflammatory disorder" refer to a condition characterized by inflammation e.g., a localized protective reaction of tissue to irritation, injury, or infection, characterized by pain, redness, swelling, and sometimes loss of function. Inflammatory diseases or disorders include e.g., allergy, asthma, and allergic rash.

As used herein, the term "immune complex-associated disease" refers to any disease characterized by the production and/or tissue deposition of immune complexes (i.e., any conjugate including an antibody and an antigen specifically bound by the antibody), including, but not limited to systemic lupus erythematosus (SLE) and related connective tissue diseases, rheumatoid arthritis, hepatitis C- and hepatitis B-related immune complex disease (e.g., cryoglobulinemia), Bechet's syndrome, autoimmune glomerulonephritides, and vasculopathy associated with the presence of LDL/anti-LDL immune complexes.

As used herein, "immunodeficiency" refers to a disease or disorder in which the subject's immune system is not functioning in normal capacity or in which it would be useful to boost a subject's immune response, for example to eliminate a tumor or cancer (e.g., tumors of the brain, lung (e.g., small cell and non-small cell), ovary, breast, prostate, colon, as well as other carcinomas and sarcomas) or an infection in a subject. The immunodeficiency can be acquired or it can be congenital.

As used herein, "graft rejection" refers to immunologically mediated hyperacute, acute, or chronic injury to a tissue or organ derived from a source other than the host. The term thus encompasses both cellular and antibody-mediated rejection, as well as rejection of both allografts and xenografts.

"Graft-versus-host disease" (GvHD) is a reaction of donated bone marrow against a patient's own tissue. GVHD is seen most often in cases where the blood marrow donor is unrelated to the patient or when the donor is related to the patient but not a perfect match. There are two forms of GVHD: an early form called acute GVHD that occurs soon after the transplant when the white cells are on the rise and a late form called chronic GVHD.

T_{H2}-mediated, atopic diseases include, but are not limited to, atopic dermatitis or eczema, eosinophilia, asthma, allergy, allergic rhinitis, and Ommen's syndrome.

As used herein, "allergy" refers to acquired hypersensitivity to a substance (allergen). Allergic conditions include eczema, allergic rhinitis or coryza, hay fever, asthma, urticaria (hives) and food allergies, and other atopic conditions

As used herein, "asthma" refers to a disorder of the respiratory system characterized by inflammation, narrowing of the airways and increased reactivity of the airways to inhaled agents. Asthma is frequently, although not exclusively associated with atopic or allergic symptoms. For example, asthma can be precipitated by exposure to an allergen, exposure to cold air, respiratory infection, and exertion.

As used herein, the terms "infection" and, equivalently, "infectious disease" refer to a condition in which an infectious organism or agent is present in a detectable amount in the blood or in a normally sterile tissue or normally sterile compartment of a subject. Infectious organisms and agents include viruses, bacteria, fungi, and parasites. The terms encompass both acute and chronic infections, as well as sepsis.

As used herein, the term "sepsis" refers to the presence of bacteria (bacteremia) or other infectious organisms or their toxins in the blood (septicemia) or in other tissue of the body.

Further provided is a compound of the invention, or a salt thereof, for use as a medicament in the treatment of the diseases or conditions described above in a mammal, for example, a human, suffering from such disease or condition. Also provided is the use of a compound of the invention, or a salt thereof, in the preparation of a medicament for the treatment of the diseases and conditions described above in a mammal, for example a human, suffering from such disorder.

This invention also encompasses pharmaceutical compositions containing a compound of the invention and methods of treating or preventing conditions and disorders by modulation of TLR7- and/or TLR8-mediated cellular activities by administering a pharmaceutical composition comprising a compound of the invention, or a salt thereof, to a patient in need thereof.

In order to use a compound of the invention or a salt thereof for the therapeutic treatment (including prophylactic treatment) of mammals including humans, it is normally formulated in accordance with standard pharmaceutical practice as a pharmaceutical composition.

According to this aspect of the invention there is provided a pharmaceutical composition that comprises a compound of the invention, or a salt thereof, as defined hereinbefore in association with a pharmaceutically acceptable diluent or carrier.

To prepare the pharmaceutical compositions according to this invention, a therapeutically or prophylactically effective amount of a compound of the invention or a salt thereof (alone or together with an additional therapeutic agent as disclosed herein) is intimately admixed, for example, with a pharmaceutically acceptable carrier according to conventional pharmaceutical compounding techniques to produce a dose. A carrier may take a wide variety of forms depending on the form of preparation desired for administration, e.g., oral or parenteral. Examples of suitable carriers include any and all solvents, dispersion media, adjuvants, coatings, antibacterial and antifungal agents, isotonic and absorption delaying agents, sweeteners, stabilizers (to promote long term storage), emulsifiers, binding agents, thickening agents, salts, preservatives, solvents, dispersion media, coatings, antibacterial and antifungal agents, isotonic and absorption delaying agents, flavoring agents, and miscellaneous materials such as buffers and absorbents that may be needed in order to prepare a particular therapeutic composition. The use of such media and agents with pharmaceutically active substances is well known in the art. Except insofar as any conventional media or agent is incompatible with a compound of the invention, its use in the therapeutic compositions and preparations is contemplated. Supplementary active ingredients can also be incorporated into the compositions and preparations as described herein.

The compositions of the invention may be in a form suitable for oral use (for example as tablets, lozenges, hard or soft capsules, aqueous or oily suspensions, emulsions, dispersible powders or granules, syrups or elixirs), for topical use (for example as creams, ointments, gels, or aqueous or oily solutions or suspensions), for administration by inhalation (for example as a finely divided powder or a liquid aerosol), for administration by insufflation (for example as a finely divided powder) or for parenteral administration (for example as a sterile aqueous or oily solution for intravenous, subcutaneous, or intramuscular dosing or as a suppository for rectal dosing). For example, compositions intended for oral use may contain, for example, one or more coloring, sweetening, flavoring and/or preservative agents.

Suitable pharmaceutically-acceptable excipients for a tablet formulation include, for example, inert diluents such as lactose, sodium carbonate, calcium phosphate or calcium carbonate, granulating and disintegrating agents such as corn starch or algenic acid; binding agents such as starch; lubricating agents such as magnesium stearate, stearic acid or talc; preservative agents such as ethyl or propyl p-hydroxybenzoate, and anti-oxidants, such as ascorbic acid. Tablet formulations may be uncoated or coated either to modify their disintegration and the subsequent absorption of the active ingredient within the gastrointestinal tract, or to improve their stability and/or appearance, in either case, using conventional coating agents and procedures well known in the art.

Compositions for oral use may be in the form of hard gelatin capsules in which the active ingredient is mixed with an inert solid diluent, for example, calcium carbonate, calcium phosphate or kaolin, or as soft gelatin capsules in which the active ingredient is mixed with water or an oil such as peanut oil, liquid paraffin, or olive oil.

Aqueous suspensions generally contain the active ingredient in finely powdered form together with one or more suspending agents, such as sodium carboxymethylcellulose, methylcellulose, hydroxypropylmethylcellulose, sodium alginate, polyvinyl-pyrrolidone, gum tragacanth and gum acacia; dispersing or wetting agents such as lecithin or condensation products of an alkylene oxide with fatty acids (for example polyoxethylene stearate), or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives (such as ethyl or propyl p-hydroxybenzoate, anti-oxidants (such as ascorbic acid), coloring agents, flavoring agents, and/or sweetening agents (such as sucrose, saccharine or aspartame).

Oily suspensions may be formulated by suspending the active ingredient in a vegetable oil (such as arachis oil, olive oil, sesame oil or coconut oil) or in a mineral oil (such as liquid paraffin). The oily suspensions may also contain a thickening agent such as beeswax, hard paraffm or cetyl alcohol. Sweetening agents such as those set out above, and flavoring agents may be added to provide a palatable oral preparation. These compositions may be preserved by the addition of an anti-oxidant such as ascorbic acid.

Dispersible powders and granules suitable for preparation of an aqueous suspension by the addition of water generally contain the active ingredient together with a dispersing or wetting agent, suspending agent and one or more preservatives. Suitable dispersing or wetting agents and suspending agents are exemplified by those already mentioned above. Additional excipients such as sweetening, flavoring and coloring agents, may also be present.

The pharmaceutical compositions of the invention may also be in the form of oil-in-water emulsions. The oily phase may be a vegetable oil, such as olive oil or arachis oil, or a mineral oil, such as for example liquid paraffin or a mixture of any of these. Suitable emulsifying agents may be, for example, naturally-occurring gums such as gum acacia or gum tragacanth, naturally-occurring phosphatides such as soya bean, lecithin, esters or partial esters derived from fatty acids and hexitol anhydrides (for example sorbitan monooleate) and condensation products of the said partial esters with ethylene oxide such as polyoxyethylene sorbitan monooleate. The emulsions may also contain sweetening, flavoring and preservative agents.

Syrups and elixirs may be formulated with sweetening agents such as glycerol, propylene glycol, sorbitol, aspartame or sucrose, and may also contain a demulcent, preservative, flavoring and/or coloring agent.

The pharmaceutical compositions may also be in the form of a sterile injectable aqueous or oily suspension, which may be formulated according to known procedures using one or more of the appropriate dispersing or wetting agents and suspending agents, which have been mentioned above. For parenteral formulations, the carrier will usually comprise sterile water, aqueous sodium chloride solution, 1,3-butanediol, or any other suitable non toxic parenterally acceptable diluent or solvent. Other ingredients including those that aid dispersion may be included. Of course, where sterile water is to be used and maintained as sterile, the compositions and carriers must also be sterilized. Injectable suspensions may also be prepared, in which case appropriate liquid carriers, suspending agents and the like may be employed.

Suppository formulations may be prepared by mixing the active ingredient with a suitable non-irritating excipient that is solid at ordinary temperatures but liquid at the rectal temperature and will therefore melt in the rectum to release the drug. Suitable excipients include, for example, cocoa butter and polyethylene glycols.

Topical formulations, such as creams, ointments, gels and aqueous or oily solutions or suspensions, may generally be obtained by formulating an active ingredient with a conventional, topically acceptable, vehicle or diluent using conventional procedures well known in the art.

Compositions for administration by insufflation may be in the form of a finely divided powder containing particles of average diameter of, for example, 30 micron or much less, the powder itself comprising either active ingredient alone or diluted with one or more physiologically acceptable carriers such as lactose. The powder for insufflation is then conveniently retained in a capsule containing, for example, 1 to 50 mg of active ingredient for use with a turbo-inhaler device, such as is used for insufflation of the known agent sodium cromoglycate.

Compositions for administration by inhalation may be in the form of a conventional pressurized aerosol arranged to dispense the active ingredient either as an aerosol containing finely divided solid or liquid droplets. Conventional aerosol propellants such as volatile fluorinated hydrocarbons or hydrocarbons may be used and the aerosol device is conveniently arranged to dispense a metered quantity of active ingredient.

Compositions for transdermal administration may be in the form of those transdermal skin patches that are well known to those of ordinary skill in the art. Other delivery systems can include time-release, delayed release or sustained release delivery systems. Such systems can avoid repeated administrations of the compounds, increasing convenience to the subject and the physician. Many types of release delivery systems are available and known to those of ordinary skill in the art. They include polymer base systems such as poly(lactide-glycolide), copolyoxalates, polycaprolactones, polyesteramides, polyorthoesters, polyhydroxybutyric acid, and polyanhydrides. Microcapsules of the foregoing polymers containing drugs are described in, for example, U.S. Patent No. 5,075,109. Delivery systems also include non-polymer systems that are: lipids including sterols such as cholesterol, cholesterol esters and fatty acids or neutral fats such as mono-di-and tri-glycerides; hydrogel release systems; silastic systems; peptide based systems; wax coatings; compressed tablets using conventional binders and excipients; partially fused implants; and the like. Specific examples include, but are not limited to: (a) erosional systems in which an agent of the invention is contained in a form within a matrix such as those described in U.S. Patent Nos. 4,452,775, 4,675,189, and 5,736,152, and (b) diffusional systems in which an active component permeates at a controlled rate from a polymer such as described in U.S. Patent Nos. 3,854,480, 5,133,974 and 5,407,686. In addition, pump-based hardware delivery systems can be used, some of which are adapted for implantation.

Compositions may be administered in the form of a solution, e.g., water or isotonic saline, buffered or unbuffered, or as a suspension, for intranasal administration as drops or as a spray. Preferably, such solutions or suspensions are isotonic relative to nasal secretions and of about the same pH, ranging e.g., from about pH 4.0 to about pH 7.4 or, from pH 6.0 to pH 7.0. Buffers should be physiologically compatible and include, simply by way of example, phosphate buffers. For example, a representative nasal decongestant is described as being buffered to a pH of about 6.2 (Remington's Pharmaceutical Sciences, Ed. By Arthur Osol, p. 1445 (1980)). Of course, the ordinary artisan can readily determine a suitable saline content and pH for an innocuous aqueous carrier for nasal administration.

Other, non-limiting examples of intranasal dosage forms containing the composition include nasal gels, creams, pastes or ointments with a viscosity of, e.g., from about 10 to about 3000 cps, or from about 2500 to 6500 cps, or greater, which may provide a more sustained contact with the nasal mucosal surfaces. Such carrier viscous formulations may be based upon, simply by way of example, polymeric carriers such as alkylcelluloses and/or other biocompatible carriers of high viscosity well known to the art (see e.g., Remington's, cited supra). The carrier containing the composition may also be soaked into a fabric material, such as gauze, that can be applied to the nasal mucosal surfaces to allow for active substances in the isolated fraction to penetrate to the mucosa.

Other ingredients, such as art known preservatives, colorants, lubricating or viscous mineral or vegetable oils, perfumes, natural or synthetic plant extracts such as aromatic oils, and humectants and viscosity enhancers such as, e.g., glycerol, can also be included to provide additional viscosity, moisture retention and a pleasant texture and odor for the formulation.

Further, for nasal administration of solutions or suspensions of the composition, various devices are available in the art for the generation of drops, droplets and sprays. For example, solutions comprising the isolated fraction can be administered into the nasal passages by means of a simple dropper (or pipet) that includes a glass, plastic or metal dispensing tube from which the contents are expelled drop by drop by means of air pressure provided by a manually powered pump, e.g., a flexible rubber bulb, attached to one end. Fine droplets and sprays can be provided by a manual or electrically powered intranasal pump dispenser or squeeze bottle as well known to the art, e.g., that is designed to blow a mixture of air and fine droplets into the nasal passages.

The amount of a compound of this invention that is combined with one or more excipients to produce a single dosage form will necessarily vary depending upon the subject treated, the severity of the disorder or condition, the rate of administration, the disposition of the compound and the discretion of the prescribing physician. However, an effective dosage is in the range of about 0.001 to about 100 mg per kg body weight per day, for example, about 0.05 to about 35 mg/kg/day, in single or divided doses. For a 70 kg human, a dosage is about 0.0005 to 2.5 g/day. For example a dosage is about 0.0005 to about 1 g/day. In some instances, dosage levels below the lower limit of the aforesaid range may be more than adequate, while in other cases still larger doses may be employed without causing any harmful side effect, provided that such larger doses are first divided into several small doses for administration throughout the day.

For further information on routes of administration and dosage regimes, see Chapter 25.3 in Volume 5 of *Comprehensive Medicinal Chemistry* (Corwin Hansch; Chairman of Editorial Board), Pergamon Press 1990, which is specifically incorporated herein by reference.

The size of the dose for therapeutic or prophylactic purposes of a compound of the invention will naturally vary according to the nature and severity of the conditions, the age and sex of the animal or patient and the route of administration, according to well known principles of medicine. It will be understood that the specific dosage level and frequency of dosage for any particular subject may be varied and will depend upon a variety of factors including the activity of the specific compound of the invention, the species, age, body weight, general health, sex and diet of the subject, the mode and time of administration, rate of excretion, drug combination, and severity of the particular condition, but can nevertheless be routinely determined by one skilled in the art.

A compound of the invention or salt thereof, is in some aspects administered to a subject in combination (e.g., in the same formulation or in separate formulations) with another therapeutic agent ("combination therapy"). The compound of the invention is administered in admixture with another therapeutic agent or is administered in a separate formulation. When administered in separate formulations, a compound of the invention and another therapeutic agent is administered substantially simultaneously or sequentially. In one aspect, a compound of the invention is administered to a subject in combination with another therapeutic agent for treating a condition or disease. In one aspect, a compound of the invention is administered to a subject in combination with another therapeutic agent for preventing a condition or disease. In one aspect, a compound of the invention is administered to a subject in combination with a vaccine for preventing a condition or disease. In one aspect, a compound of the invention is administered to a subject in combination with an infectious disease vaccine. In one aspect, a compound of the invention is administered to a subject in combination with a cancer vaccine.

A compound of the invention may also be helpful in individuals having compromised immune function. For example, a compound of the invention may be used for treating or preventing the opportunistic infections and tumors that occur after suppression of cell mediated immunity in, for example, transplant patients, cancer patients and HIV patients.

Such combination treatment may involve, in addition to a compound of the invention, conventional surgery or radiotherapy or chemotherapy. Such chemotherapy may include one or more of the following categories of anti-tumor agents: (i) antiproliferative/antineoplastic drugs and combinations thereof; (ii) cytostatic agents; (iii) agents which inhibit cancer cell invasion; (iv) inhibitors of growth factor function; (v) antiangiogenic agents; (vi) vascular damaging agents; (vii) antisense therapies; (viii) gene therapy approaches; (ix) interferon; and (x) immunotherapy approaches.

Therapeutic agents for treating or preventing respiratory diseases which may be administered in combination with a compound of the invention in a subject method include, but are not limited to beta adrenergics which include bronchodilators including albuterol, isoproterenol sulfate, metaproterenol sulfate, terbutaline sulfate, pirbuterol acetate and sahneterol formotorol; steroids including beclomethasone dipropionate, flunisolide, fluticasone, budesonide and triamcinolone acetonide. Anti-inflammatory drugs used in connection with the treatment or preventing of respiratory diseases include steroids such as beclomethasone dipropionate, triamcinolone acetonide, flunisolide and fluticasone. Other anti-inflammatory drugs include cromoglycates such as cromolyn sodium. Other respiratory drugs which would qualify as bronchodilators include anticholenergics including ipratropium bromide. Antihistamines include, but are not limited to, diphenhydramine, carbinoxamine, clemastine, dimenhydrinate, pryilamine, tripelennamine, chlorpheniramine, brompheniramine, hydroxyzine, cyclizine, meclizine, chlorcyclizine, promethazine, doxylamine, loratadine, and terfenadine. Particular anti¬histamines include rhinolast (Astelin®), claratyne (Claritin®), claratyne D (Claritin D®), telfast (Allegra®), Zyrtec®, and beconase.

In some embodiments, a compound of the invention is administered as a combination therapy with interferon-gamma (IFN-gamma), a corticosteroid such as prednisone, prednisolone, methyl prednisolone, hydrocortisone, cortisone, dexamethasone, betamethasone, etc., or a combination thereof, for the treatment or preventing of interstitial lung disease, e.g., idiopathic pulmonary fibrosis.

In some embodiments, a compound of the invention is administered in combination therapy with a known therapeutic agent used in the treatment of cystic fibrosis ("CF"). Therapeutic agents used in the treatment of CF include, but are not limited to, antibiotics; anti-inflammatory agents; DNAse (e.g., recombinant human DNAse; pulmozyme; dornase alfa); mucolytic agents (e.g., N-acetylcysteine; Mucomyst™; Mucosil™); decongestants; bronchodilators (e.g., theophylline; ipatropium bromide); and the like.

In some embodiments, a compound of the invention is administered prophylatically for the prevention of cardiovascular disease e.g., atherosclerosis.

In another embodiment of the invention, an article of manufacture, or "kit", containing materials useful for the treatment or prevention of the diseases described above is provided.

In one embodiment, the kit comprises a container comprising a composition of the invention, or pharmaceutically acceptable salt thereof. In one embodiment, the invention provides a kit for treating or preventing a TLR7- and/or TLR8-mediated disorder. In another embodiment, the invention provides a kit for a condition or disorder treatable by selective modulation of the immune system in a subject. The kit may further comprise a label or package insert on or associated with the container. Suitable containers include, for example, bottles, vials, syringes, blister pack, etc. The container may be formed from a variety of materials such as glass or plastic. The container holds a compound of the invention or a pharmaceutical formulation thereof in an amount effective for treating or preventing the condition, and may have a sterile access port (for example, the container may be an intravenous solution bag or a vial having a stopper pierceable by a hypodermic injection needle). The label or package insert indicates that the composition is used for treating or preventing the condition of choice. In one embodiment, the label or package inserts indicates that the composition comprising a compound of the invention can be used, for example, to treat or prevent a disorder treatable by modulation of TLR7- and/or TLR8-mediated cellular activities. The label or package insert may also indicate that the composition can be used to treat or prevent other disorders. Alternatively, or additionally, the kit may further comprise a second container comprising a pharmaceutically acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

The kit may further comprise directions for the administration of the compound of the invention and, if present, the second pharmaceutical formulation. For example, if the kit comprises a first composition comprising a compound of the.invention and a second pharmaceutical formulation, the kit may further comprise directions for the simultaneous, sequential or separate administration of the first and second pharmaceutical compositions to a patient in need thereof.

In another embodiment, the kits are suitable for the delivery of solid oral forms of a compound of the invention, such as tablets or capsules. Such a kit includes, for example, a number of unit dosages. Such kits can include a card having the dosages oriented in the order of their intended use. An example of such a kit is a "blister pack". Blister packs are well known in the packaging industry and are widely used for packaging pharmaceutical unit dosage forms. If desired, a memory aid can be provided, for example in the form of numbers, letters, or other markings or with a calendar insert, designating the days in the treatment schedule in which the dosages can be administered.

According to one embodiment, the kit may comprise (a) a first container with a compound of the invention contained therein; and optionally (b) a second container with a second pharmaceutical formulation contained therein, wherein the second pharmaceutical formulation comprises a second compound which may be effective in treating or preventing a condition or disorder by selective modulation of TLR7- and/or TLR8-mediated cellular activities. Alternatively, or additionally, the kit may further comprise a third container comprising a pharmaceutically acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

In certain other embodiments wherein the kit comprises a pharmaceutical formulation of a compound of the invention and a second formulation comprising a second therapeutic agent, the kit may comprise a container for containing the separate formulations, such as a divided bottle or a divided foil packet; however, the separate compositions may also be contained within a single, undivided container. Typically, the kit comprises directions for the administration of the separate components. The kit form is particularly advantageous when the separate components are administered in different dosage forms (e.g., oral and parenteral), are administered at different dosage intervals, or when titration of the individual components of the combination is desired by the prescribing physician.

Throughout the description, where compositions are described as having, including, or comprising specific components, it is contemplated that compositions also consist essentially of, or consist of, the recited components. Similarly, where methods or processes are described as having, including, or comprising specific process steps, the processes also consist essentially of, or consist of, the recited processing steps. Further, it should be understood that the order of steps or order for performing certain actions is immaterial so long as the invention remains operable. Moreover, two or more steps or actions can be conducted simultaneously.

The synthetic processes of the invention can tolerate a wide variety of functional groups; therefore various substituted starting materials can be used. The processes generally provide the desired final compound at or near the end of the overall process, although it may be desirable in certain instances to further convert the compound to a pharmaceutically acceptable salt, ester or prodrug thereof.

Compounds of the present invention can be prepared in a variety of ways using commercially available starting materials, compounds known in the literature, or from readily prepared intermediates, by employing standard synthetic methods and procedures either known to those skilled in the art, or which will be apparent to the skilled artisan in light of the teachings herein. Standard synthetic methods and procedures for the preparation of organic molecules and functional group transformations and manipulations can be obtained from the relevant scientific literature or from standard textbooks in the field. Although not limited to any one or several sources, classic texts such as Smith, M. B., March, J., March's Advanced Organic Chemistry: Reactions, Mechanisms, and Structure, 5th edition, John Wiley & Sons: New York, 2001; and Greene, T.W., Wuts, P.G. M., Protective Groups in Organic Syntheses, 3rd edition, John Wiley & Sons: New York, 1999, incorporated by reference herein, are useful and recognized reference textbooks of organic synthesis known to those in the art. The following descriptions of synthetic methods are designed to illustrate, but not to limit, general procedures for the preparation of compounds of the present invention.

Compounds of the present invention can be conveniently prepared by a variety of methods familiar to those skilled in the art. The compounds of this invention with each of the formulae described herein may be prepared according to the following procedures from commercially available starting materials or starting materials which can be prepared using literature procedures. These procedures show the preparation of representative compounds of this invention.

Compounds designed, selected and/or optimized by methods described above, once produced, can be characterized using a variety of assays known to those skilled in the art to determine whether the compounds have biological activity. For example, the molecules can be characterized by conventional assays, including but not limited to those assays described below, to determine whether they have a predicted activity, binding activity and/or binding specificity.

Furthermore, high-throughput screening can be used to speed up analysis using such assays. As a result, it can be possible to rapidly screen the molecules described herein for activity, using techniques known in the art. General methodologies for performing high-throughput screening are described, for example, in Devlin (1998) High Throughput Screening, Marcel Dekker; and U.S. Patent No. 5,763,263. High-throughput assays can use one or more different assay techniques including, but not limited to, those described below.

All publications and patent documents cited herein are incorporated herein by reference as if each such publication or document was specifically and individually indicated to be incorporated herein by reference. Citation of publications and patent documents is not intended as an admission that any is pertinent prior art, nor does it constitute any admission as to the contents or date of the same. The invention having now been described by way of written description, those of skill in the art will recognize that the invention can be practiced in a variety of embodiments and that the foregoing description and examples below are for purposes of illustration and not limitation of the claims that follow.

### EXAMPLES

In order to illustrate the invention, the following examples are included. However, it is to be understood that these examples do not limit the invention and are only meant to suggest a method of practicing the invention.

### Example 1 Synthetic Procedures

### Synthesis of Compound 63

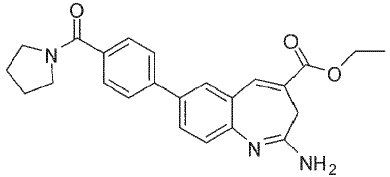

### (1E,4E)-Ethyl 2-amino-7-(4-(pyrrolidine-1-carbonyl)phenyl)-3H-benzo[b]azepine-4-carboxylate.

Step A: Potassium nitrate (49.2 g, 0.486 mol) was added to 240 g of cooled sulfuric acid in a three neck round bottom flask, keeping the temperature below 25 °C. This was followed by the slow addition of 3-bromobenzaldehyde (30.0 g, 0.162 mol). Once the addition was complete, the mixture was allowed to gradually warm to room temperature overnight. The mixture was then poured into 500 mLs of ice water, resulting in a light yellow precipitate. The solids were collected by filtration and dried under vacuum for several hours. Purification of the crude product was done in the following way: The collected solids were divided into two lots and each lot purified using two 340 g Biotage Snap Cartridges in series with 3:1 Hexanes:EtOAc as the eluant. Obtained 20 g of 5-bromo-2-nitrobenzaldehyde (54%) as a light yellow solid. ¹H NMR (400 MHz, CDCl₃) δ 10.42 (s, 1H), 8.07, (d, 1H), 8.03, (d, 1H), 7.89 (dd, 1H).

Step B: α-Cyanomethylcarboethoxyethylidene (37 g, 0.096 mol) and 5-bromo-2-nitrobenzaldehyde (20 g, 0.087 mol) were combined in 400 mLs of dry toluene and brought to reflux. After 10 hours, the mixture was allowed to cool to room temperature, and then concentrated under reduced pressure. The resulting crude material was divided into two lots and each lot purified using two 340 g Biotage Snap Cartridges in series with 3:1 Hexanes:EtOAc as the eluant. Obtained 22.9 g (78%) of (E)-ethyl 3-(5-bromo-2-nitrophenyl)-2-(cyanomethyl)acrylate as a light yellow solid. ¹H NMR (400 MHz, CDCl₃) δ 8.13-8.16 (m, 2H), 7.77 (dd, 1H), 7.59 (d, 1H), 4.39 (q, 1H), 3.34 (s, 2H), 1.40 t, 3H).

Step C: (E)-ethyl 3-(5-bromo-2-nitrophenyl)-2-(cyanomethyl)acrylate (22.0 g, 0.065 mol) was taken up in 250 mLs of acetic acid and the mixture was warmed to 80°C, resulting in a solution. To this was added iron powder (21.7 g, 0.389 mol) and the mixture stirred at 80 °C for two hours, during which time the mixture became a thick slurry. The mixture was then allowed to cool to room temperature, and then filtered. The collected solids were rinsed with EtOAc, and the filtrate concentrated under reduced pressure. The resulting dark crude material was taken up in 25% IPA/DCM (∼ 500 mLs) and the residual acetic acid was quenched with 1M aq. sodium carbonate (∼ 500 mLs). This material was transferred to a 2 liter separatory funnel, and upon separation of the organics/aqueous material, a yellow precipitate formed in the organic layer. The organics were isolated and the solids collected by filtration and dried under high vacuum overnight to give 13.6 g of (1E,4E)-ethyl 2-amino-7-bromo-3H-benzo[b]azepine-4-carboxylate as a light yellow solid (lot 1). The filtrate was then dried over sodium sulfate and concentrated to give 6 g of a more yellow/orange solid (lot 2 - not as clean as lot 1 by HPLC/NMR). ¹H NMR (400 MHz, DMSO-d₆) δ 7.70 (m, 1H), 7.64 (d, 1H), 7.39 (dd, 1H), 6.95-6.99 (m, 3H), 4.24 (q, 2H), 2.86 (s, 2H), 1.29 (t, 3H); *m*/*z* (APCI-pos) M+1 = 309.1, 311.1.

Step D: (1E,4E)-Ethyl 2-amino-7-bromo-3H-benzo[b]azepine-4-carboxylate (0.150 g, 0.485 mmol), 4-(pyrrolidine-1-carbonyl)phenylboronic acid (0.181 g, 0.825 mmol), Pd(PPh₃)₄ (0.056 g, 0.0458 mmol), and 2M aqueous potassium carbonate (0.728 mls, 1.46 mmol) were combined in 4 mls of acetonitrile in a microwave reaction vial. This was heated to 100°C for 45 minutes in the microwave. The mixture was diluted with EtOAc, washed with brine, dried and concentrated. 100g Snap Cartridge Biotage (7% MeOH/DCM/0.5% NH₄OH) afforded 80 mgs (41%) of (1E,4E)-ethyl 2-amino-7-(4-(pyrrolidine-1-carbonyl)phenyl)-3H-benzo[b]azepine-4-carboxylate as an orange solid. ¹H NMR (400 MHz, CDCl₃) δ 7.86-7.88 (m, 1H), 7.59-7.67 (m, 6H), 7.32-7.36 (m, 1H), 4.29-4.37 (m, 2H), 3.64-3.72 (m, 2H), 3.47-3.54 (m, 2H), 3.03 (s, 2H), 1.86-2.01 (m, 4H), 1.36-1.43 (m, 3H); *mlz* (APCI-pos) M+1 = 404.2.

### Synthesis of Compound 33

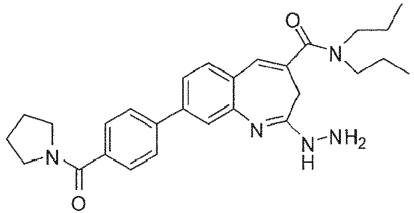

### (1E,4E)-2-Hydrazinyl-N,N-dipropyl-8-(4-(pyrrolidine-1-carbonyl)phenyl)-3H-benzo[b]azepine-4-carboxamide

Step A: Preparation of (E)-1-(4-bromo-2-nitrostyryl)pyrrolidine: A solution of 4-bromo-2-nitrotoluene (100 g, 463 mmol), pyrrolidine (46.2 mL, 565 mmol), and N,N-dimethylformamide dimethylacetal (75.6 mL, 565 mmol) was refluxed for 4 hours at 110 °C. The reaction mixture was cooled to room temperature and concentrated under reduced pressure to give the crude (E)-1-(4-bromo-2-nitrostyryl)pyrrolidine that was used directly without further purification.

Step B: Preparation of 4-bromo-2-nitrobenzaldehyde: To a solution of sodium periodate (298 g, 1.40 mol) in THF-H₂O (4 L, 1:1) at 0 °C was added (E)-1-(4-bromo-2-nitrostyryl)pyrrolidine (138 g, 464 mmol). The mixture was stirred for 15 h and then filtered to remove solid precipitates. The aqueous layer from the filtrate was separated and extracted with EtOAc (4 x 200 mL). The combined organic layers were washed with H₂O (2 x 200 mL), dried over MgSO₄, filtered, and concentrated under reduced pressure to give the crude product that was purified by silica gel flash column chromatography (5% EtOAc in hexanes) to afford 91 g (86%) of 4-bromo-2-nitrobenzaldehyde.

Step C: Preparation of 3-nitro-4'-(pyrrolidine-1-carbonyl)biphenyl-4-carbaldehyde: To a solution of 4-bromo-2-nitrobenzaldehyde (20.2 g, 87.9 mmol), 4-(pyrrolidine-1-carbonyl)phenylboronic acid (21.2 g, 96.7 mmol), and Pd(PPh₃)₄ (508 mg, 0.440 mmol) in toluene (200 mL) was added EtOH (40 mL) followed by Na₂CO₃ (70.0 mL, 140 mmol, 2 M aq solution) at room temperature. The resulting mixture was heated at 100 °C for 18 h. The reaction mixture was cooled to room temperature and the organic layer was separated. The aqueous layer was extracted with EtOAc (300 mL). The combined organic layers were washed with brine (500 mL), dried over MgSO₄, filtered, and concentrated under reduced pressure to give the crude material that was combined with another batch of the crude material obtained from an additional run in the same reaction scale. The combined crude material was purified by silica gel flash column chromatography (CH₂Cl₂ to 1% MeOH in CH₂Cl₂) to afford 51 g (90%) of 3-nitro-4'-(pyrrolidine-1-carbonyl)biphenyl-4-carbaldehyde.

Step D: Preparation of (E)-ethyl2-(cyanomethyl)-3-(3-nitro-4'-(pyrrolidine-1-carbonyl)biphenyl-4-yl)acrylate: A mixture of 3-nitro-4'-(pyrrolidine-1-carbonyl)biphenyl-4-carbaldehyde (20.0 g, 61.7 mmol) and α-cyanomethylcarboethoxyethylidene triphenylphosphorane (26.3 g, 67.8 mmol) in toluene (200 mL) was gently refluxed for 2.5 h. The reaction mixture was cooled to room temperature and concentrated under reduced pressure to give the crude (E)-ethyl 2-(cyanomethyl)-3-(3-nitro-4'-(pyrrolidine-1-carbonyl)biphenyl-4-yl)acrylate that was used directly without further purification.

Step E: Preparation of (1E,4E)-ethyl 2-amino-8-(4-(pyrrolidine-1-carbonyl)phenyl)-3H-benzo[b]azepine-4-carboxylate: To a solution of the crude (E)-ethyl 2-(cyanomethyl)-3-(3-nitro-4'-(pyrrolidine-1-carbonyl)biphenyl-4-yl)acrylate in AcOH (650 mL) was added iron (29.1 g, 521 mmol) at room temperature. The resulting mixture was heated at 85 °C for 4 h. The reaction mixture was cooled to room temperature and diluted with CH₂Cl₂ (250 mL). The solids were filtered off and washed with CH₂Cl₂ (200 mL). The filtrate was concentrated under reduced pressure to give the crude material that was diluted with CH₂Cl₂ (250 mL) again. To this mixture was slowly added sat'd aq Na₂CO₃ (∼330 mL) with vigorous stirring until it became basic (pH ∼9-10). The resulting mixture was filtered off and washed with CH₂Cl₂ (∼250 mL). The aqueous layer was separated and extracted with CH₂Cl₂ (2 x 150 mL). The combined organic layers were washed with brine, dried over MgSO₄, and filtered to give the crude material that was diluted with EtOAc (70 mL). The mixture was kept for 16 h at room temperature. The suspension was filtered. The solids filtered off were washed with EtOAc (100 mL) to give the crude product that was washed with a small amount of CH₂Cl₂ to afford 20 g (62% based on 95% purity) of (1E,4E)-ethyl 2-amino-8-(4-(pyrrolidine-1-carbonyl)phenyl)-3H-benzo[b]azepine-4-carboxylate.

Step F: Preparation of(1E,4E)-ethyl 2-(tert-butoxycarbonylamino)-8-(4-(pyrrolidine-1-carbonyl)phenyl)-3H-benzo[b]azepine-4-carboxylate: To a mixture of (1E,4E)-ethyl 2-amino-8-(4-(pyrrolidine-1-carbonyl)phenyl)-3H-benzo[b]azepine-4-carboxylate (9.60 g, 23.8 mmol) in CH₂Cl₂ (100 mL) was added Boc₂O (5.97 mg, 27.4 mmol) at room temperature. The reaction mixture was stirred for 3 days. The resulting mixture was washed with sat'd aq NaHCO₃ and brine. The organic layer was separated and dried over MgSO₄, filtered, and concentrated under reduced pressure to give 12.7 g of the crude (1E,4E)-ethyl 2-(tert-butoxycarbonylamino)-8-(4-(pyrrolidine-1-carbonyl)phenyl)-3H-benzo[b]azepine-4-carboxylate that was used directly without further purification. MS APCI(+) m/z 504 (M+1) detected.

Step G: Preparation of (1E,4E)-2-(tert-butoxycarbonylamino)-8-(4-(pyrrolidine-1-carbonyl)phenyl)-3H-benzo[b]azepine-4-carboxylic acid: To a solution of (1E,4E)-ethyl 2-(tert-butoxycarbonylamino)-8-(4-(pyrrolidine-1-carbonyl)phenyl)-3H-benzo[b]azepine-4-carboxylate (12.0 g, 23.8 mmol) in THF-EtOH (60 mL/60 mL) was added 4 N aq. LiOH (23.8 mL, 95.3 mmol) at 0 °C. The reaction mixture was warmed to room temperature and stirred for 21 h. Additional 6 mL of 4 N aq LiOH was added twice after 21 h and 24 h. After stirring for additional 6 h, the resulting mixture was concentrated under reduced pressure to give the crude material that was diluted with water (50 mL) and acidified to a pH of ∼3.5 with 1 N aq phosphoric acid (∼450 mL). ∼250 mL of CH₂Cl₂ was added during acidification to extract the crude product out of the sticky suspension. The solids formed during acidification were filtered off using a glass filter packed with Celite. The aqueous layer was separated and extracted with CH₂Cl₂ (3 x 100 mL). The combined organic layers were dried over MgSO₄, filtered, and concentrated under reduced pressures to give 10.2 g (90%) of the crude (1E,4E)-2-(tert-butoxycarbonylamino)-8-(4-(pyrrolidine-1-carbonyl)phenyl)-3H-benzo[b]azepine-4-carboxylic acid that was used directly without further purification. MS APCI(+) m/z 476 (M+1) detected.

Step H: (1E,4E)-2-(tert-butoxycarbonylamino)-8-(4-(pyrrolidine-1-carbonyl)phenyl)-3H-benzo[b]azepine-4-carboxylic acid (0.5 g, 1.05 mmol), HOBT (0.213 g, 1.58 mmol), and EDCI (0.213 g, 1.58 mmol) were taken up in 10 mLs of dichloromethane and stirred at room temperature for 1 hour. Dipropylamine (0.216 mLs, 1.58 mmol) and triethylamine (0.293 mLs, 2.103 mmol) were then added and the mixture stirred for one hour, then diluted with dichloromethane, washed with saturated ammonium chloride, brine, dried over sodium sulfate and concentrated. Biotage purification (50 g Snap cartridge, 1:1 EtOAc:Hexanes) afforded 0.2 g (34%) of tert-butyl (1E,4E)-4-(dipropylcarbamoyl)-8-(4-(pyrrolidine-1-carbonyl)phenyl)-3H-benzo[b]azepin-2-ylcarbamate. *m*/*z* (APCI-pos) M+1 = 558.9.

Step I: tert-Butyl (1E,4E)-4-(dipropylcarbamoyl)-8-(4-(pyrrolidine-1-carbonyl)phenyl)-3H-benzo[b]azepin-2-ylcarbamate (0.075 g, 0.134 mmol) was dissolved in 1.5 mLs of ethanol in a reaction vial. To this solution was added hydrazine (0.0215 mLs, 0.671 mmol), vial sealed and the mixture heated to 80°C for 30 minutes. The mixture was then diluted with EtOAc, washed twice with 1M aqueous sodium carbonate, water, dried over sodium sulfate and concentrated under reduced pressure. Preparative thin layer chromatography (0.5 mm plate, 5% MeOH/DCM/0.5% NH₄OH) afforded (1E,4E)-2-hydrazinyl-N,N-dipropyl-8-(4-(pyrrolidine-1-carbonyl)phenyl)-3H-benzo[b]azepine-4-carboxamide (39%) as a yellow solid. ¹H NMR (400 MHz, DMSO-d₆) δ 7.59-7.66 (m, 5H), 7.28-7.31 (m, 2H), 6.76 (s, 1H), 3.63-3.71 (m, 4H), 3.47-3.52 (m, 2H), 3.29-3.42 (m, 4H), 1.87-2.02 (m, 4H), 1.56-1.68 (m, 4H), 0.80-0.97 (m, 6H); *m*/*z* (APCI-pos) M+1 = 474.2.

### Synthesis of Compound 76

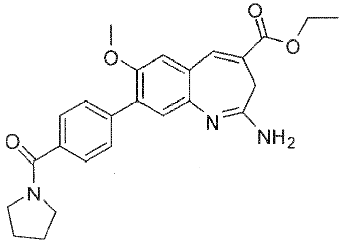

### (1E,4E)-ethyl 2-amino-7-methoxy-8-(4-(pyrrolidine-1-carbonyl)phenyl)-3H-benzo[b]azepine-4-carboxylate

Step A: To a solution of 4-(benzyloxy)-3-methoxybenzaldehyde (2.00 g, 8.090 mmol) in 1,2-dichloroethane (8 mL) at -30°C was slowly added fuming nitric acid (4.00 ml, 88.21 mmol) while maintaining the temperature at -15°C for 3 hours. The reaction mixture was poured into water and extracted with EtOAc (2 x 25 mL). The combined organic layers were dried over MgSO₄, filtered, and concentrated under reduced pressure to give the crude material that was triturated with a mixture solvents of EtOAc and hexanes to afford 1.81 g (78%) of 4-(benzyloxy)-5-methoxy-2-nitrobenzaldehyde. The filtrate was concentrated again under reduced pressure to give additional 615 mg (24% based on 91% purity) of the desired product. A total of 2.37 g of the product was obtained after consideration of the purity. The product appeared to be a mixture of the desired product and the over-nitrated product on the O-benzyl group. Both batches were used directly without further purification.

Step B: A mixture of 4-(benzyloxy)-5-methoxy-2-nitrobenzaldehyde (1.81 g, 6.30 mmol) in TFA (11 mL) was heated at 60°C for 20 hours then refluxed for 5 hours. The reaction mixture was concentrated under reduced pressure to give the crude material that was purified by silica gel flash column chromatography (0.5% MeOH in CH₂Cl₂) to afford 236 mg (19%) of 4-hydroxy-5-methoxy-2-nitrobenzaldehyde.

Step C: To a solution of 4-hydroxy-5-methoxy-2-nitrobenzaldehyde (0.2358 g, 1.196 mmol) and 1,1,1-trifluoro-N-phenyl-N-(trifluoromethylsulfonyl)methanesulfonamide (0.5341 g, 1.495 mmol) in CH₂Cl₂ (2.5 mL) was added TEA (0.2513 ml, 1.794 mmol) at room temperature. The reaction mixture became dark red and was stirred for 23 hours at room temperature. The reaction mixture was diluted with CH₂Cl₂ (25 mL) and washed with saturated aqueous NaHCO₃ (15 mL) followed by brine (15 mL). The organic layer was dried over MgSO₄, filtered, and concentrated under reduced pressure to give the crude material that was purified by silica gel flash column chromatography (5 to 10% EtOAc in hexanes) to afford 224 mg (57%) of 4-formyl-2-methoxy-5-nitrophenyl trifluoromethanesulfonate.

Step D: Ethyl 2-(cyanomethyl)-3-(5-methoxy-2-nitro-4-(trifluoromethylsulfonyloxy) phenyl)acrylate (81%) was prepared according to Synthesis of Compound 47, Step D, substituting 4-formyl-2-methoxy-5-nitrophenyl trifluoromethanesulfonate for 3-nitro-4'-(pyrrolidine-1-carbonyl)biphenyl-4-carbaldehyde.

Step E: (1E,4E)-Ethyl 2-amino-7-methoxy-8-(trifluoromethylsulfonyloxy)-3H-benzo[b]azepine-4-carboxylate (49%) was prepared according to Synthesis of Compound 47, Step E, substituting ethyl 2-(cyanomethyl)-3-(5-methoxy-2-nitro-4-(trifluoromethylsulfonyloxy) phenyl)acrylate for (E)-ethyl 2-(cyanomethyl)-3-(3-nitro-4'-(pyrrolidine-1-carbonyl) biphenyl-4-yl)acrylate. *m*/*z* (APCI-pos) M+1 = 409.0.

Step F: To a vial charged with (1E,4E)-ethyl 2-amino-7-methoxy-8-(trifluoromethylsulfonyloxy)-3H-benzo[b]azepine-4-carboxylate (0.107 g, 0.262 mmol), 4-(pyrrolidine-1-carbonyl)phenylboronic acid (0.117 g, 0.524 mmol), Pd(OAc)₂ (0.00600 g, 0.0262 mmol), 4,4'-(phenylphosphinidene)bisbenzenesulfonic acid dipotassium hydrate (0.0289 g, 0.0524 mmol), Na₂CO₃ (0.0842 g, 0.786 mmol), and a magnetic stirring bar was added MeCN-H₂O (2.5 mL/1.2 mL). The reaction mixture was bubbled with N₂ for 1 min and was heated for 2 hours at 65°C. The reaction mixture was cooled to room temperature and the solids materials were filtered off. The filtrate was extracted with EtOAc (3x15 mL). The combined organic layers were dried over MgSO₄, filtered, and concentrated under reduced pressure to give the crude material that was purified by silica gel flash column chromatography (3 to 7% MeOH in CH₂Cl₂, gradient) to afford the desired product that still contained the boronic acid. The mixture was dissolved into CH₂Cl₂ (15 mL) again, washed with saturated aqueous Na₂CO₃ (3 x 20 mL), dried over MgSO₄, filtered, and concentrated under reduced pressure to afford 59 mg (52%) of (1E,4E)-ethyl 2-amino-7-methoxy-8-(4-(pyrrolidine-1-carbonyl)phenyl)-3H-benzo[b] azepine-4-carboxylate. ¹H NMR (400 MHz, CDCl₃) δ 7.80 (s, 1H), 7.60-7.63 (m, 2H), 7.55-7.58 (m, 2H), 7.24 (s, 1H), 6.91 (s, 1H), 4.88 (br s, 2H), 4.29-4.37 (m, 2H), 3.83 (s, 3H), 3.64-3.69 (m, 2H), 3.50-3.55 (m, 2H), 2.97 (s, 2H), 1.85-2.01 (m, 4H), 1.36-1.42 (m, 3H); *m*/*z* (APCI-pos) M+1 = 434.2.

### Synthesis of Compounds 12 and 65

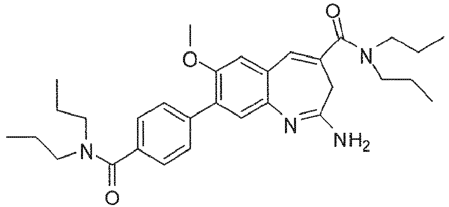

### (1E,4E)-2-Amino-8-(4-(dipropylcarbamoyl)phenyl)-7-methoxy-N,N-dipropyl-3H-benzo[b]azepine-4-carboxamide And

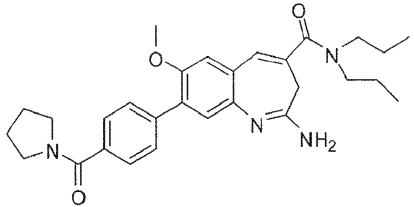

### (1E,4E)-2-Amino-7-methoxy-N,N-dipropyl-8-(4-(pyrrolidine-1-carbonyl)phenyl)-3H-benzo[b]azepine-4-carboxamide

Step A: (1E,4E)-2-Amino-7-methoxy-N,N-dipropyl-8-(4-(pyrrolidine-1-carbonyl) phenyl)-3H-benzo[b]azepine-4-carboxamide (20%) was prepared according to the synthesis of Compound 70, Step E, substituting (1E,4E)-ethyl 2-amino-7-methoxy-8-(4-(pyrrolidine-1-carbonyl)phenyl)-3H-benzo[b] azepine-4-carboxylate (Compound 76) for(1E,4E)-ethyl 2-amino-7-methoxy-3H-benzo[b]azepine-4-carboxylate. ¹H NMR (400 MHz, CDCl₃) δ 7.54-7.62 (m, 4H), 7.28 (s, 1H), 6.79-6.82 (m, 2H), 3.82 (s, 3H), 3.64-3.70 (m, 2H), 3.39-3.55 (m, 6H), 2.89 (s, 2H), 1.85-2.01 (m, 4H), 1.61-1.71 (m, 4H), 0.87-0.98 (m, 6H); m/z (APCI-pos) M+1 = 489.2. (1E,4E)-2-Amino-8-(4-(dipropylcarbamoyl)phenyl)-7-methoxy-N,N-dipropyl-3H-benzo[b]azepine-4-carboxamide (19%) was also isolated from the reaction mixture. ¹H NMR (400 MHz, CDCl₃) δ 7.55-7.60 (m, 2H), 7.37-7.41 (m, 2H), 7.33 (s, 1H), 6.81-6.84 (m, 2H), 3.82 (s, 3H), 3.38-3.53 (m, 6H), 3.20-3.29 (m, 2H), 2.98 (s, 2H), 1.52-1.77 (m, 8H), 0.75-1.03 (m, 12H); *m*/*z* (APCI-pos) M+1 = 519.3.

### Synthesis of Compound 74

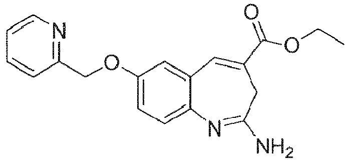

### (1E,4E)-ethyl 2-amino-7-(pyridin-2-ylmethoxy)-3H-benzo[b]azepine-4-carboxylate

Step A: 5-Hdroxy-2-nitrobenzaldehyde (7.44 g, 44.5 mmol) was dissolved in 60 mls of DMF. To this solution was added potassium carbonate (13.0 g, 93.5 mmol), resulting in an orange-red mixture. After stirring at room temperature for 5 minutes, 2-chloromethylpyridine hydrochloride (6.25 g, 49.0 mmol) was then added and the mixture was warmed to 65°C for 16 hours. The reaction mixture was then concentrated under reduced pressure and the resulting crude material was taken up in dichloromethane, washed with water, saturated sodium bicarbonate solution, brine, dried over sodium sulfate and concentrated under reduced pressure. Obtained 10.45 g (91%) of 2-nitro-5-(pyridin-2-ylmethoxy)benzaldehyde.

Step B: (E)-Ethyl 2-(cyanomethyl)-3-(2-nitro-5-(pyridin-2-ylmethoxy)phenyl)acrylate (99%) was prepared according to Synthesis of Compound 63, Step B, substituting 2-nitro-5-(pyridin-2-ylmethoxy)benzaldehyde for 5-bromo-2-nitrobenzaldehyde.

Step C: (1E,4E)-ethyl 2-amino-7-(pyridin-2-ylmethoxy)-3H-benzo[b]azepine-4-carboxylate (19%) was prepared according to the synthesis of Compound 63, Step C, substituting (E)-ethyl 2-(cyanomethyl)-3-(2-nitro-5-(pyridin-2-ylmethoxy)phenyl)acrylate for (E)-ethyl 3-(5-bromo-2-nitrophenyl)-2-(cyanomethyl)acrylate. ¹H NMR (400 MHz, MeOH-d₄) δ 8.55 (d, 1H), 7.88 (t, 1H), 7.74 (s, 1H), 7.63 (d, 1H), 7.38 (t, 1H), 7.05-7.13 (m, 2H), 7.01-7.03 (m, 1H), 5.19 (s, 2H), 4.29 (q, 2H), 2.96 (s, 2H), 1.36 (t, 3H).

### Synthesis of Compound 88

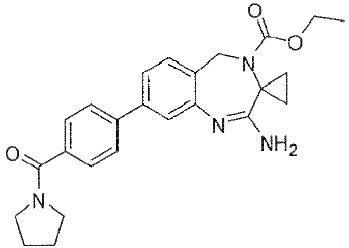

### (E)-Ethyl 2-amino-8-(4-(pyrrolidine-1-carbonyl)phenyl)spiro[benzo[e][1,4]diazepine-3,1'-cyclopropane]-4(5H)-carboxylate

Step A: Step A: Preparation of (E)-1-(4-bromo-2-nitrostyryl)pyrrolidine: A solution of 4-bromo-2-nitrotoluene (100 g, 463 mmol), pyrrolidine (46.2 mL, 565 mmol), and N,N-dimethylformamide dimethylacetal (75.6 mL, 565 mmol) was refluxed for 4 hours at 110 °C. The reaction mixture was cooled to room temperature and concentrated under reduced pressure to give the crude (E)-1-(4-bromo-2-nitrostyryl)pyrrolidine that was used directly without further purification.

Step B: Preparation of 4-bromo-2-nitrobenzaldehyde: To a solution of sodium periodate (298 g, 1.40 mol) in THF-H₂O (4 L, 1:1) at 0 °C was added (E)-1-(4-bromo-2-nitrostyryl)pyrrolidine (138 g, 464 mmol). The mixture was stirred for 15 h and then filtered to remove solid precipitates. The aqueous layer from the filtrate was separated and extracted with EtOAc (4 x 200 mL). The combined organic layers were washed with H₂O (2 x 200 mL), dried over MgSO₄, filtered, and concentrated under reduced pressure to give the crude product that was purified by silica gel flash column chromatography (5% EtOAc in hexanes) to afford 91 g (86%) of 4-bromo-2-nitrobenzaldehyde.

Step C: Preparation of 3-nitro-4'-(pyrrolidine-1-carbonyl)biphenyl-4-carbaldehyde: To a solution of 4-bromo-2-nitrobenzaldehyde (20.2 g, 87.9 mmol), 4-(pyrrolidine-1-carbonyl)phenylboronic acid (21.2 g, 96.7 mmol), and Pd(PPh₃)₄ (508 mg, 0.440 mmol) in toluene (200 mL) was added EtOH (40 mL) followed by Na₂CO₃ (70.0 mL, 140 mmol, 2 M aq solution) at room temperature. The resulting mixture was heated at 100 °C for 18 h. The reaction mixture was cooled to room temperature and the organic layer was separated. The aqueous layer was extracted with EtOAc (300 mL). The combined organic layers were washed with brine (500 mL), dried over MgSO₄, filtered, and concentrated under reduced pressure to give the crude material that was combined with another batch of the crude material obtained from an additional run in the same reaction scale. The combined crude material was purified by silica gel flash column chromatography (CH₂Cl₂ to 1% MeOH in CH₂Cl₂) to afford 51 g (90%) of 3-nitro-4'-(pyrrolidine-1-carbonyl)biphenyl-4-carbaldehyde.

Step D: 3-Nitro-4'-(pyrrolidine-1-carbonyl)biphenyl-4-carbaldehyde (0.410 g, 1.27 mmol) was dissolved in 10 mls of methanol. To this was added 1-aminocyclopropanecarbonitrile hydrochloride (0.150 g, 1.27 mmol) followed by sodium cyanoborohydride (0.0954 g. 1.52 mmol) and the mixture was stirred at room temperature for 16 hours. The mixture was then concentrated under reduced pressure and the resulting residue was taken up in EtOAc, washed twice with saturated sodium bicarbonate, dried over sodium sulfate and concentrated to 0.145 g of crude 1-((3-nitro-4'-(pyrrolidine-1-carbonyl)biphenyl-4-yl)methylamino)cyclopropanecarbonitrile (29%). *m*/*z* (APCI-pos) M+1 = 391.2.

Step E: 1-((3-Nitro-4'-(pyrrolidine-1-carbonyl)biphenyl-4-yl)methylamino) cyclopropanecarbonitrile (0.100 g, 0.256 mmol) was dissolved in 3 mls of dry dichloromethane and chilled to 0°C. To this was added pyridine (0.0518 mls, 0.640 mmol) followed by ethyl chloroformate (0.0488 mls, 0.512 mmol) and the mixture was then allowed to warm to room temperature over 16 hours. The mixture was then diluted with DCM (50 mls), washed once with 1N aqueous HCl, saturated sodium bicarbonate, dried over sodium sulfate and concentrated. Flash chromatography (Flash 40 Biotage 40M cartridge, 1:1 EtOac:Hexane to 100% EtOAc) afforded 0.045 g (38%) of ethyl 1-cyanocyclopropyl((3-nitro-4'-(pyrrolidine-1-carbonyl)biphenyl-4-yl)methyl)carbamate. *m*/*z* (APCI-pos) M+1 = 463.3.

Step F: 1-Cyanocyclopropyl((3-nitro-4'-(pyrrolidine-1-carbonyl)biphenyl-4-yl)methyl) carbamate (0.040 g, 0.0865 mmol) was dissolved in 3 mls of acetic acid. To this was added iron powder (0.0241 g, 0.432 mmol) and the mixture was warmed to 90°C for 30 minutes. The mixture was allowed to cool to room temperature and then poured into saturated sodium bicarbonate solution (100 mls), followed by the addition of 50 mls of EtOAc. This mixture was then filtered through GF/F filter paper, organics isolated, dried over sodium sulfate and concentrated under reduced pressure. Preparative thin layer chromatography (2 x 0.5 mm plates, 10% MeOH/DCM/0.5% NH₄OH) afforded 12 mgs (32%) of (E)-ethyl 2-amino-8-(4-(pyrrolidine-1-carbonyl)phenyl)spiro[benzo[e] [1,4]diazepine-3,1'-cyclopropane]-4(5H)-carboxylate. ¹H NMR (400 MHz, CDCl₃) δ 7.56-7.66 (m, 4H), 7.21-7.29 (m, 3H), 4.56 (s, 2H), 4.21 (q, 2H), 3.64-3.70 (m, 2H), 3.46-3.53 (m, 2H), 1.86-2.01 (m, 4H), 4.31 (t, 3H), 1.08-1.12 (m, 2H), 0.95-1.00 (m, 2H); *m*/*z* (APCI-pos) M+1 = 433.2.

### Synthesis of Compound 47

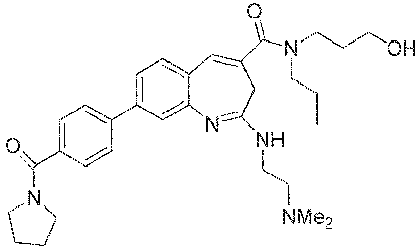

### (1E,4E)-2-(2-(Dimethylamino)ethylamino)-N-(3-hydroxypropyl)-N-propyl-8-(4-(pyrrolidine-1-carbonyl)phenyl)-3H-benzo[b]azepine-4-carboxamide

Step A: Preparation of (E)-1-(4-bromo-2-nitrostyryl)pyrrolidine: A solution of 4-bromo-2-nitrotoluene (100 g, 463 mmol), pyrrolidine (46.2 mL, 565 mmol), and N,N-dimethylformamide dimethylacetal (75.6 mL, 565 mmol) was refluxed for 4 hours at 110 °C. The reaction mixture was cooled to room temperature and concentrated under reduced pressure to give the crude (E)-1-(4-bromo-2-nitrostyryl)pyrrolidine that was used directly without further purification.

Step B: Preparation of 4-bromo-2-nitrobenzaldehyde: To a solution of sodium periodate (298 g, 1.40 mol) in THF-H₂O (4 L, 1:1) at 0 °C was added (E)-1-(4-bromo-2-nitrostyryl)pyrrolidine (138 g, 464 mmol). The mixture was stirred for 15 h and then filtered to remove solid precipitates. The aqueous layer from the filtrate was separated and extracted with EtOAc (4 x 200 mL). The combined organic layers were washed with H₂O (2 x 200 mL), dried over MgSO₄, filtered, and concentrated under reduced pressure to give the crude product that was purified by silica gel flash column chromatography (5% EtOAc in hexanes) to afford 91 g (86%) of 4-bromo-2-nitrobenzaldehyde.

Step C: Preparation of 3-nitro-4'-(pyrrolidine-1-carbonyl)biphenyl-4-carbaldehyde: To a solution of 4-bromo-2-nitrobenzaldehyde (20.2 g, 87.9 mmol), 4-(pyrrolidine-1-carbonyl)phenylboronic acid (21.2 g, 96.7 mmol), and Pd(PPh₃)₄ (508 mg, 0.440 mmol) in toluene (200 mL) was added EtOH (40 mL) followed by Na₂CO₃ (70.0 mL, 140 mmol, 2 M aq solution) at room temperature. The resulting mixture was heated at 100 °C for 18 h. The reaction mixture was cooled to room temperature and the organic layer was separated. The aqueous layer was extracted with EtOAc (300 mL). The combined organic layers were washed with brine (500 mL), dried over MgSO₄, filtered, and concentrated under reduced pressure to give the crude material that was combined with another batch of the crude material obtained from an additional run in the same reaction scale. The combined crude material was purified by silica gel flash column chromatography (CH₂Cl₂ to 1% MeOH in CH₂Cl₂) to afford 51 g (90%) of 3-nitro-4'-(pyrrolidine-1-carbonyl)biphenyl-4-carbaldehyde.

Step D: Preparation of (E)-ethyl 2-(cyanomethyl)-3-(3-nitro-4'-(pyrrolidine-1-carbonyl)biphenyl-4-yl)acrylate: A mixture of 3-nitro-4'-(pyrrolidine-1-carbonyl)biphenyl-4-carbaldehyde (20.0 g, 61.7 mmol) and α-cyanomethylcarboethoxyethylidene triphenylphosphorane (26.3 g, 67.8 mmol) in toluene (200 mL) was gently refluxed for 2.5 h. The reaction mixture was cooled to room temperature and concentrated under reduced pressure to give the crude (E)-ethyl 2-(cyanomethyl)-3-(3-nitro-4'-(pyrrolidine-1-carbonyl)biphenyl-4-yl)acrylate that was used directly without further purification.

Step E: Preparation of (1E,4E)-ethyl 2-amino-8-(4-(pyrrolidine-1-carbonyl)phenyl)-3H-benzo[b]azepine-4-carboxylate: To a solution of the crude (E)-ethyl 2-(cyanomethyl)-3-(3-nitro-4'-(pyrrolidine-1-carbonyl)biphenyl-4-yl)acrylate in AcOH (650 mL) was added iron (29.1 g, 521 mmol) at room temperature. The resulting mixture was heated at 85 °C for 4 h. The reaction mixture was cooled to room temperature and diluted with CH₂Cl₂ (250 mL). The solids were filtered off and washed with CH₂Cl₂ (200 mL). The filtrate was concentrated under reduced pressure to give the crude material that was diluted with CH₂Cl₂ (250 mL) again. To this mixture was slowly added sat'd aq Na₂CO₃ (∼330 mL) with vigorous stirring until it became basic (pH ∼9-10). The resulting mixture was filtered off and washed with CH₂Cl₂ (∼250 mL). The aqueous layer was separated and extracted with CH₂Cl₂ (2 x 150 mL). The combined organic layers were washed with brine, dried over MgSO₄, and filtered to give the crude material that was diluted with EtOAc (70 mL). The mixture was kept for 16 h at room temperature. The suspension was filtered. The solids filtered off were washed with EtOAc (100 mL) to give the crude product that was washed with a small amount of CH₂Cl₂ to afford 20 g (62% based on 95% purity) of (1E,4E)-ethyl 2-amino-8-(4-(pyrrolidine-1-carbonyl)phenyl)-3H-benzo[b]azepine-4-carboxylate.

Step F: Preparation of (1E,4E)-ethyl 2-(tert-butoxycarbonylamino)-8-(4-(pyrrolidine-1-carbonyl)phenyl)-3H-benzo[b]azepine-4-carboxylate: To a mixture of (1E,4E)-ethyl 2-amino-8-(4-(pyrrolidine-1-carbonyl)phenyl)-3H-benzo[b]azepine-4-carboxylate (9.60 g, 23.8 mmol) in CH₂Cl₂ (100 mL) was added Boc₂O (5.97 mg, 27.4 mmol) at room temperature. The reaction mixture was stirred for 3 days. The resulting mixture was washed with sat'd aq NaHCO₃ and brine. The organic layer was separated and dried over MgSO₄, filtered, and concentrated under reduced pressure to give 12.7 g of the crude (1E,4E)-ethyl 2-(tert-butoxycarbonylamino)-8-(4-(pyrrolidine-1-carbonyl)phenyl)-3H-benzo[b]azepine-4-carboxylate that was used directly without further purification. MS APCI(+) m/z 504 (M+1) detected.

Step G: Preparation of (1E,4E)-2-(tert-butoxycarbonylamino)-8-(4-(pyrrolidine-1-carbonyl)phenyl)-3H-benzo[b]azepine-4-carboxylic acid: To a solution of (1E,4E)-ethyl 2-(tert-butoxycarbonylamino)-8-(4-(pyrrolidine-1-carbonyl)phenyl)-3H-benzo [b] azepine-4-carboxylate (12.0 g, 23.8 mmol) in THF-EtOH (60 mL/60 mL) was added 4 N aq. LiOH (23.8 mL, 95.3 mmol) at 0 °C. The reaction mixture was warmed to room temperature and stirred for 21 h. Additional 6 mL of 4 N aq LiOH was added twice after 21 h and 24 h. After stirring for additional 6 h, the resulting mixture was concentrated under reduced pressure to give the crude material that was diluted with water (50 mL) and acidified to a pH of ∼3.5 with 1 N aq phosphoric acid (∼450 mL). ∼250 mL of CH₂Cl₂ was added during acidification to extract the crude product out of the sticky suspension. The solids formed during acidification were filtered off using a glass filter packed with Celite. The aqueous layer was separated and extracted with CH₂Cl₂ (3 x 100 mL). The combined organic layers were dried over MgSO₄, filtered, and concentrated under reduced pressures to give 10.2 g (90%) of the crude (1E,4E)-2-(tert-butoxycarbonylamino)-8-(4-(pyrrolidine-1-carbonyl)phenyl)-3H-benzo[b]azepine-4-carboxylic acid that was used directly without further purification. MS APCI(+) m/z 476 (M+1) detected.

Step H: 3-(Propylamino)propan-1-ol (7.80 g, 66.6 mmol) and triethylamine (11.1 mls, 79.9 mmol) were dissolved in 600 mls of dichloromethane and chilled to 0°C. To this mixture was added TBDMSCI (11.0 g, 73.2 mmol) and the mixture was allowed to gradually warm to room temperature over a 16 hour period. The mixture was then washed with saturated sodium bicarbonate solution (3X), dried over sodium sulfate and concentrated to 16 g (quantitative) of 3-(tert-butyldimethylsilyloxy)-N-propylpropan-1-amine.

Step I: To a slurry of (1E,4E)-2-(tert-butoxycarbonylamino)-8-(4-(pyrrolidine-1-carbonyl)phenyl)-3H-benzo[b]azepine-4-carboxylic acid (0.100 g, 0.210 mmol) and HOBT (0.0426 g, 0.315 mmol) in CH₂Cl₂ (1 mL) was added EDCI (0.0605 g, 0.315 mmol) at room temperature. The reaction mixture was stirred for 50 min. To this mixture were added 3-(tert-butyldimethylsilyloxy)-N-propylpropan-1-amine (0.0690 ml, 0.252 mmol) and TEA (0.0586 ml, 0.421 mmol) at room temperature. The resulting mixture was stirred for 1.5 h. The reaction mixture was diluted with EtOAc (10 mL) and was washed with sat'd aq NH₄Cl (5 mL). The organic layer was separated. The aqueous layer was extracted with EtOAc (10 mL) again. The combined organic layers were washed with brine (5 mL), saturated aqueous NaHCO₃ (5 mL), and brine (5 mL). The organic layer was dried over MgSO₄, filtered, and concentrated under reduced pressure to give ∼168 mg of the crude tert-butyl (1E,4E)-4-((3-(tert-butyldimethylsilyloxy)propyl)(propyl)carbamoyl)-8-(4-(pyrrolidine-1-carbonyl)phenyl)-3H-benzo[b]azepin-2-ylcarbamate. *m*/*z* (APCI-pos) M+1 = 689.1.

Step J: A mixture of tert-butyl (1E,4E)-4-((3-(tert-butyldimethylsilyloxy)propyl) (propyl)carbamoyl)-8-(4-(pyrrolidine-1-carbonyl)phenyl)-3H-benzo[b]azepin-2-ylcarbamate (0.075 g, 0.109 mmol), N1,N1-dimethylethane-1,2-diamine (0.0250 ml, 0.218 mmol), and TEA (0.040 ml, 0.286 mmol) in DMF (2 mL) was heated at 65 °C for 2.5 h in a sealed vial. Additional 0.020 mL (0.17 mmol) of N1,N1-dimethylethane-1,2-diamine was added. The resulting mixture was heated at 65 °C for additional 2.5 h. The reaction mixture was cooled to room temperature, diluted with EtOAc (15 mL), and washed with brine (2 x 15 mL). The organic layer was dried over MgSO₄, filtered, and concentrated under reduced pressure to give the crude (1E,4E)-N-(3-(tert-butyldimethylsilyloxy)propyl)-2-(2-(dimethylamino)ethylamino)-N-propyl-8-(4-(pyrrolidine-1-carbonyl)phenyl)-3H-benzo[b]azepine-4-carboxamide (*m*/*z* (APCI-pos) M+1 = 660.4) that was used directly without further purfication.

Step K: To a solution of (1E,4E)-N-(3-(tert-butyldimethylsilyloxy)propyl)-2-(2-(dimethylamino)ethylamino)-N-propyl-8-(4-(pyrrolidine-1-carbonyl)phenyl)-3H-benzo[b]azepine-4-carboxamide (0.0718 g, 0.109 mmol) in THF (2 mL) was added HCl (4 M in dioxane) (0.0952 ml, 0.381 mmol) at room temperature. The reaction mixture was stirred for 2 h at room temperature. The reaction mixture was diluted with EtOAc (15 mL) and washed with saturated aqueous NaHCO₃ (10 mL). The aqueous layer was separated and extracted with EtOAc (2 x 15 mL). The combined organic layers were washed with sat'd aq NaHCO3 (4 x 10 mL), dried over MgSO₄, filtered, and concentrated under reduced pressure to give the crude material that was purified by silica gel flash column chromatography (5% MeOH in CH₂Cl₂ to a mixture solution of NH₄OH-MeOH-CH₂Cl₂ (1/5/95)) to afford 21 mg (36% for two steps) of (1E,4E)-2-(2-(dimethylamino)ethylamino)-N-(3-hydroxypropyl)-N-propyl-8-(4-(pyrrolidine-1-carbonyl)phenyl)-3H-benzo[b]azepine-4-carboxamide (36%). ¹H NMR (400 MHz, CDCl₃) δ 7.68-7.72 (m, 2H), 7.58-7.62 (m, 2H), 7.50-7.53 (m, 1H), 7.30-7.33 (m, 1H), 7.23-7.27 (m, 1H), 6.84 (s, 1H), 3.58-3.71 (m, 6H), 3.40-3.53 (m, 6H), 2.84 (s, 2H), 2.47-2.54 (m, 2H), 2.27 (s, 6H), 1.94-2.02 (m, 2H), 1.87-1.93 (m, 2H), 1.87-1.93 (m, 2H), 1.77-1.85 (m, 2H), 1.61-1.72 (m, 2H), 0.84-0.94 (m, 3H); *m*/*z* (APCI-pos) M+1 = 546.3.

### Synthesis of Compound 90

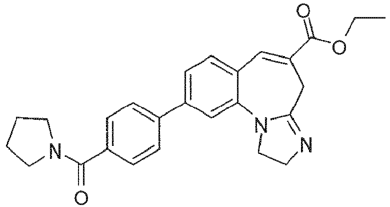

### (E)-ethyl 9-(4-(pyrrolidine-1-carbonyl)phenyl)-2,4-dihydro-1H-benzo[f]imidazo[1,2-a]azepine-5-carboxylate

Step A: To a solution of 4-bromo-1-methyl-2-nitrobenzene (300 g, 1.38 mol) in acetic anhydride (2400 mL) at 0 °C, was added slowly concentrated sulfuric acid (324 ml), followed by a solution of chromium trioxide (384 g, 3.84 mole) in acetic anhydride (2160 ml). The internal temperature was controlled below 10 °C. After stirring for 1h, the contents in the flask were poured into a mixture of ice and water. The solid was filtered and washed with water until the washings were colorless. The product was suspended in 1800 ml of 2% aqueous sodium carbonate solution with stirring. After thorough mixing, the solid was filtered and washed with water and dried under reduced pressure.

A suspension of the diacetate in a mixture of 1360 ml of concentrated hydrochloric acid, 1250 ml of water, and 480 ml of ethanol was refluxed for 45 minutes. The mixture was then cooled to room temperature and the solid was filtered and washed with water. 4-bromo-2-nitrobenzaldehyde (147 g, 45% for two steps) was afforded as a brown solid without further purification.

Step B: A mixture of the 4-bromo-2-nitrobenzaldehyde (25.45g, 0.11mol) and α-cyanomethylcarboethoxyethylidene (50g, 0.129mol) in toluene (800mL) was gently refluxed for 2.5 hours. The reaction mixture was cooled to room temperature and concentrated under reduced pressure to give the crude (E)-ethyl 3-(4-bromo-2-nitrophenyl)-2-(cyanomethyl)acrylate that was used directly without further purification.

Step C: To a solution of the crude (E)-ethyl 3-(4-bromo-2-nitrophenyl)-2-(cyanomethyl) acrylate in AcOH (500 mL) was added iron (40 g, 0.716 mol) at room temperature. The resulting mixture was heated at 85 °C for 6 hours. The reaction mixture was cooled to room temperature and diluted with CH₂Cl₂. The resulting mixture was filtered and the solids were washed with CH₂Cl₂. The filtrate was concentrated under reduced pressure to give viscous oil. To the crude material was added CH₂Cl₂ and aqueous Na₂CO₃ was added slowly with stirring until its pH became 9-10. The mixture was filtered off and washed with CH₂Cl₂. The organic layer was separated. The aqueous layer was extracted with CH₂Cl₂. The combined organic layers were washed with brine, dried over anhydrous Na₂SO₄. The solvent was concentrated under reduced pressure to give the crude material that was purified by silica gel flash column chromatography (PE 100% to PE/EA 2/1) to afford 20 g (58.8% for two steps) of (1E,4E)-ethyl 2-amino-8-bromo-3H-benzo[b]azepine-4-carboxylate and (E)-ethyl 8-bromo-2-oxo-2,3-dihydro-1H-benzo[b]azepine-4-carboxylate (2g).

Step D: A solution of (E)-ethyl 8-bromo-2-oxo-2,3-dihydro-1H-benzo[b]azepine-4-carboxylate (0.25g, 1.0mmol) and Lawesson's reagent (0.45g, 1.1 mmol) in dioxane (20mL) was refluxed for 16 hours. The reaction mixture was concentrated and purified via flash chromatography (PE 100% to PET:EA =5:1) to afford 0.149 g (58%) of (E)-ethyl 8-bromo-2-thioxo-2,3-dihydro-1H-benzo[b]azepine-4-carboxylate.

Step E: To the solution of (E)-ethyl 8-bromo-2-thioxo-2,3-dihydro-1H-benzo[b]azepine-4-carboxylate (2 g, 8 mmol) and 2-bromoethanamine hydrobromide (11.5 g, 88 mmol) in 500 ml THF added HgCl₂ (2.3 g, 8 mmol) at 80°C. The mixture was refluxed for 1 hour. The THF was removed under reduced pressure and residue was suspended in DCM. The solid was removed by filter, and then the organic layer was washed with 0.2 M aqueous Na₂S₂O₃ to remove the unreacted HgCl₂. After drying over K₂CO₃, the organic layer was evaporated under reduced pressure. The crude compound was purified by silica gel column (from 1:1 PET:EA to 5:1 DCM: MeOH) affording 1.8 g (86.9%) of (E)-ethyl 9-bromo-2,4-dihydro-1H-benzo[f]imidazo[1,2-a]azepine-5-carboxylate.

Step F: Under the nitrogen atmosphere, (E)-ethyl 9-bromo-2,4-dihydro-1H-benzo[f]imidazo[1,2-a]azepine-5-carboxylate (0.41g, 10mmol), 4-(pyrrolidine-1-carbonyl)phenylboronic acid (0.44g, 20mmol), Cs₂CO₃ (0.61g, 20mmol) and Pd(PPh₃)₄ (0.1g, 10mol%) were dissolved in 50ml, EtOH. The mixture was refluxed until completion indicated by TLC (usually 2 hrs). After cooling, the reaction mixture was poured into water and extracted with EtOAc. The combined organic layer was dried over MgSO₄, concentrated in vacuo, the crude product was purified by chromatography silica gel (EA 100% to 1:10 MeOH:EA) to afford 0.3g, (60%) of (E)-ethyl 9-(4-(pyrrolidine-1-carbonyl)phenyl)-2,4-dihydro-1H-benzo[f]imidazo[1,2-a]azepine-5-carboxylate. ¹H NMR (400 MHz, CDCl₃) δ 7.79 (s, 1H), 7.62-7.64 (m, 4H), 7.38-7.41 (m, 1H), 7.14-7.16 (m, 1H), 4.28-4.34 (m, 2H), 3.97-4.03 (m, 2H), 3.85-3.91 (m, 2H), 3.65-3.70 (m, 2H), 3.47-3.52 (m, 4H), 1.87-2.02 (m, 4H), 1.35-1.40 (m, 3H); *m*/*z* (APCI-pos) M+1 = 430.3.

### Synthesis of Compound 46

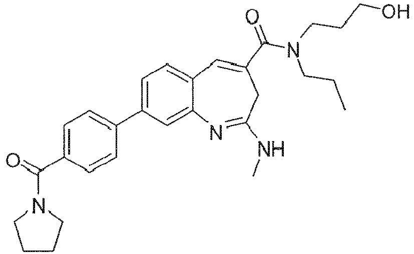

### (1E,4E)-N-(3-Hydroxypropyl)-2-(methylamino)-N-propyl-8-(4-(pyrrolidine-1-carbonyl)phenyl)-3H-benzo[b]azepine-4-carboxamide

Step A: tert-Butyl (1E,4E)-4-((3-(tert-butyldimethylsilyloxy)propyl)(propyl)carbamoyl)-8-(4-(pyrrolidine-1-carbonyl)phenyl)-3H-benzo[b]azepin-2-ylcarbamate (59%) was prepared according to the Synthesis of Compound33, Step H, substituting 3-(tert-butyldimethylsilyloxy)-N-propylpropan-1-amine for dipropylamine. *m*/*z* (APCI-pos) M+1 = 689.0.

Step B: A mixture of tert-butyl (1E,4E)-4-((3-(tert-butyldimethylsilyloxy)propyl)-(propyl)carbamoyl)-8-(4-(pyrrolidine-1-carbonyl)phenyl)-3H-benzo[b]azepin-2-ylcarbamate (0.145 g, 0.210 mmol), methanamine (2 M in THF) (0.210 ml, 0.421 mmol), and TEA (0.0590 ml, 0.421 mmol) in DMF (2 mL) was heated at 65 °C for 1.5 h in a sealed vial. Additional 0.11 mL (0.22 mmol) of MeNH₂ was added and the resulting mixture was heated at 65°C for additional 3 h. The reaction mixture was cooled to room temperature, diluted with EtOAc (15 mL), and washed with sat'd aq NaHCO₃ followed by brine. The organic layer was dried over MgSO₄, filtered, and concentrated under reduced pressure to give the crude material that was purified by silica gel flash column chromatography (1 to 5% MeOH in CH₂Cl₂) to afford 48 mg (38% for two steps) of (1E,4E)-N-(3-(tert-butyldimethylsilyloxy)propyl)-2-(methylamino)-N-propyl-8-(4-(pyrrolidine-1-carbonyl)phenyl)-3H-benzo[b]azepine-4-carboxamide. *m*/*z* (APCI-pos) M+1 = 603.3.

Step C: To a solution of (1E,4E)-N-(3-(tert-butyldimethylsilyloxy)propyl)-2-(methylamino)-N-propyl-8-(4-(pyrrolidine-1-carbonyl)phenyl)-3H-benzo[b]azepine-4-carboxamide (0.045 g, 0.0746 mmol) in THF (2 mL) was added HCl (4M in dioxane) (0.0467 ml, 0.187 mmol) at room temperature. The reaction mixture was stirred for 1 h at room temperature. The reaction mixture was diluted with ether (10 mL) and washed with sat'd aq. NaHCO₃ (3 x 10 mL). The organic layer was dried over MgSO₄, filtered, and concentrated under reduced pressure to give the crude material that was purified by silica gel flash column chromatography (5 to 7% MeOH in CH₂Cl₂) to afford 19 mg (53%) of (1E,4E)-N-(3-hydroxypropyl)-2-(methylamino)-N-propyl-8-(4-(pyrrolidine-1-carbonyl)-phenyl)-3H-benzo[b]azepine-4-carboxamide. ¹H NMR (400 MHz, CDCl₃) δ 7.71 (d, 2H), 7.60 (d, 2H), 7.56 (m, 1H), 7.30-7.35 (m, 1H), 7.25-7.29 (m, 1H), 6.85 (s, 1H), 4.92 (br s, 1H), 3.57-3.71 (m, 6H), 3.44-3.54 (m, 4H), 2.98 (s, 3H), 2.79 (s, 2H), 1.80-2.03 (m, 6H), 1.58 -1.75 (m, 2H), 0.89-0.96 (m, 3H); *m*/*z* (APCI-pos) M+1 = 489.2.

### Synthesis of Compound 70

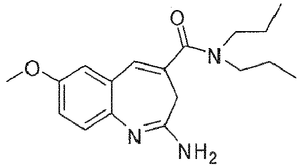

### (1E,4E)-2-amino-7-methoxy-N,N-dipropyl-3H-benzo[b]azepine-4-carboxamide

Step A: (E)-1-(5-Methoxy-2-nitrostyryl)pyrrolidine (100%) was prepared according to Synthesis of Compound 47, Step A, substituting 4-methoxy-2-methyl-1-nitrobenzene for 4-bromo-2-nitrotoluene, and used without further purification.

Step B: 5-Methoxy-2-nitrobenzaldehyde (97%) was prepared according to Synthesis of Compound 47, Step B, substituting (E)-1-(5-methoxy-2-nitrostyryl)pyrrolidine for (E)-1-(4-bromo-2-nitrostyryl)pyrrolidine, and used without further purification.

Step C: (E)-Ethyl 2-(cyanomethyl)-3-(5-methoxy-2-nitrophenyl)acrylate (100%) was prepared according to Synthesis of Compound 47, Step D, substituting 5-methoxy-2-nitrobenzaldehyde for 3-nitro-4'-(pyrrolidine-1-carbonyl)biphenyl-4-carbaldehyde, and used without further purification.

Step D: (1E,4E)-Ethyl 2-amino-7-methoxy-3H-benzo[b]azepine-4-carboxylate (60%) was prepared according to Synthesis of Compound 47, Step E, substituting (E)-ethyl 2-(cyanomethyl)-3-(5-methoxy-2-nitrophenyl)acrylate for (E)-ethyl 2-(cyanomethyl)-3-(3-nitro-4'-(pyrrolidine-1-carbonyl)biphenyl-4-yl)acrylate. *m*/*z* (APCI-pos) M+1 = 261.1.

Step E: To a solution of dipropylamine (0.105 ml, 0.768 mmol) in toluene (2 mL) at 0 °C was added AlMe₃ (2M in toluene) (0.960 ml, 1.92 mmol). The resulting mixture was warmed to room temperature. To this mixture was added portionwise (1E,4E)-ethyl 2-amino-7-methoxy-3H-benzo[b]azepine-4-carboxylate (0.100 g, 0.384 mmol). The reaction mixture was heated at 100 °C for 21 h. The reaction mixture was cooled to room temperature and poured onto 0.5 N aq. Rochelle's salt. The resulting mixture was vigorously stirred for 20 min and extracted with EtOAc (3 x 20 mL). The combined organic layers were dried over MgSO₄, filtered, and concentrated under reduced pressure to give the crude material that was purified by silica gel flash column chromatography (3 to 9% MeOH in CH₂Cl₂, gradient) to afford 46 mg (38%) of (1E,4E)-2-amino-7-methoxy-N,N-dipropyl-3H-benzo[b]azepine-4-carboxamide. ¹H NMR (400 MHz, CDCl₃) δ 7.16-7.19 (m, 1H), 6.93-6.96 (m, 1H), 6.72-6.76 (m, 2H), 4.82 (br s, 2H), 3.83 (s, 3H), 3.37-3.49 (m, 4H), 2.75 (s, 2H), 1.60-1.70 (m, 4H), 0.86-0.97 (m, 6H); *m*/*z* (APCI-pos) M+1 = 316.2.

### Synthesis of Compound 25

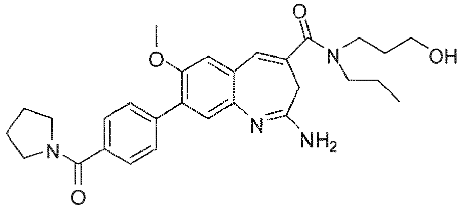

### (1E,4E)-2-Amino-N-(3-hydroxypropyl)-7-methoxy-N-propyl-8-(4-(pyrrolidine-1-carbonyl)phenyl)-3H-benzo[b]azepine-4-carboxamide

Step A: (1E,4E)-2-Amino-N-(3-hydroxypropyl)-7-methoxy-N-propyl-8-(4-(pyrrolidine-1-carbonyl)phenyl)-3H-benzo[b]azepine-4-carboxamide (25%) was prepared according to Synthesis of Compound 70, Step E, substituting 3-(propylamino)propan-1-ol for dipropylamine and (1E,4E)-ethyl 2-amino-7-methoxy-8-(4-(pyrrolidine-1-carbonyl)phenyl)-3H-benzo[b] azepine-4-carboxylate (Compound 76) for (1E,4E)-ethyl 2-amino-7-methoxy-3H-benzo[b]azepine-4-carboxylate. ¹H NMR (400 MHz, CDCl₃) δ 7.54-7.63 (m, 4H), 7.24 (s, 1H), 6.83 (s, 1H), 6.80 (s, 1H), 4.90 (br s, 2H), 3.81 (s, 3H), 3.58-3.70 (m, 6H), 3.44-3.55 (m, 4H), 2.81 (s, 2H), 1.80-2.00 (m, 6H), 1.66-1.75 (m, 2H), 0.82-0.97 (m, 3H); *m*/*z* (APCI-pos) M+1 = 505.2.

### Example 2

### HEK/TLR assays

The activity of the compounds of this invention may be determined by the following assays.

The HEK-293 hTLR transfectant assay employs HEK293 cells stably transfected with various hTLRs and transiently co-transfected with a plasmid containing an NF-κB driven secreted embryonic alkaline phosphate (SEAP) reporter gene. Stimulation of TLRs activates their downstream signaling pathways and induces nuclear translocation of the transcription factor NF-κB. Reporter gene activity is then measured using a spectrophotometric assay.

To measure agonist activity, human embryonic kidney (HEK) cells (e.g., 293XL-hTLR8 cells available from InvivoGen, San Diego, CA) are prepared according to supplier's instructions and incubated with various concentrations of test compound overnight. The amount of induced luciferase is measured by reading the absorbance at 650 mu. Agonist compounds of the invention have an MC₅₀ of 25 µM or less, wherein MC₅₀ is defined as the concentration at which 50% of maximum induction is seen.

For the TLR8 antagonist assays, cells are transiently transfected with the reporter gene on Day 1 per the supplier's instructions. Antagonist compounds are added to the cultures on Day 2 followed by addition of a TLR8 agonist approximately 2 hours later. Cultures are incubated overnight and SEAP activity is measured on Day 3.

In a typical assay, 50,000 HEK239 hTLR8 cells are seeded per culture well and transiently transfected with the SEAP reporter gene. Antagonists are added to cultures in culture medium and > 1% DMSO over a concentration range of 0.1 nanomolar to 10 micromolar. TLR8 agonists are added to cultures 2 hours later at a fixed concentration (e.g., 1 micromolar or 10 micromolar of Compound A) and cultures are then incubated for 16-24 hrs at 37°C in a humidified CO incubator. Antagonists are also evaluated for activity in the absence of agonist.

TLR8 agonist Compound A has the structure:

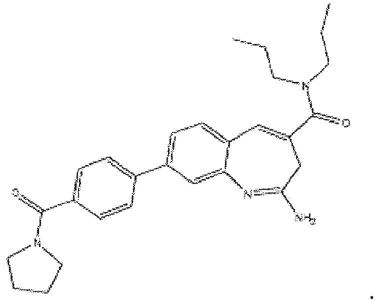

See WO 2007/024612.

TLR8 antagonist activity at 25 µM is presented in Table 2, where + denotes a % inhibition of 20-39, ++ denotes a % inhibition of 40-59, +++ denotes a % inhibition of 60-79 and ++++ denotes a % inhibition of 80-99. In some cases, antagonist activity was assessed at lower concentrations, for example, at 8.3, 2.8, or less than 1 µM.

**Table 2**

| **Compound** | **% inhibition** |
|---|---|
| 47 | + |
| 88 | + |
| 90 | +++ |
| 76 | +++ |
| 33 | ++++ |
| 63 | ++++ |
| 74 | +++ |
| 46 | ++ |
| 70 | ++ |
| 25 | + |
| 65 | +++ |
| 76 | ++++ |

TLR8 antagonist activity was measured in a hTLR8 assay format, measuring IC₅₀ values. Compounds were incubated with hTLR8 reporter cells for two hours, then 1 µM Compound A was added to induce TLR8 overnight. IC₅₀ₛ were then calculated.

IC₅₀ Results are shown below in Table 3, where + indicates an IC₅₀ of greater than or equal to 10 µM, ++ indicates a value of 5-10, +++ indicates a value of 1-5, and ++++ indicates a value of less than 1.

**Table 3**

| **Compound** | **IC₅₀ (µM)** |
|---|---|
| 88 | ++ |
| 76 | + |
| 33 | +++ |
| 63 | ++++ |
| 74 | +++ |
| 65 | ++ |
| 12 | +++ |

IC₅₀ Results are shown below in Table 4, where + indicates an IC₅₀ (nM) of greater than or equal to 10,000, ++ indicates a value of 1,000-10,000, +++ indicates a value of less than 1,000.

**Table 4**

| | **IC50, nM, vs.** | | | | |
|---|---|---|---|---|---|
| | **VTX-378** | | | **3M002** | |
| **Compound No.** | **0.5 µM** | **1 µM** | **10 µM** | **1 µM** | **10 µM** |
| 3173 | +++ | +++ | | | |
| 3348 | +++ | +++ | | | +++ |
| 3260 | +++ | +++ | | | +++ |
| 2931 | | +++ | ++ | | |
| 2984 | | +++ | ++ | | |
| 2986 | | +++ | + | | |
| 2987 | | +++ | ++ | | |
| 2966 | | + | + | | |
| 2919 | | + | + | | |
| 2976 | | + | + | | |
| 3000 | | + | + | | |
| 2922 | | ++ | + | | |
| 2929 | | + | + | | |
| 2962 | | + | + | | |
| 2926 | | ++ | + | | |
| 2954 | | + | + | | |
| 3020 | | + | + | | |

### Example 3

### Human PBMCs assays

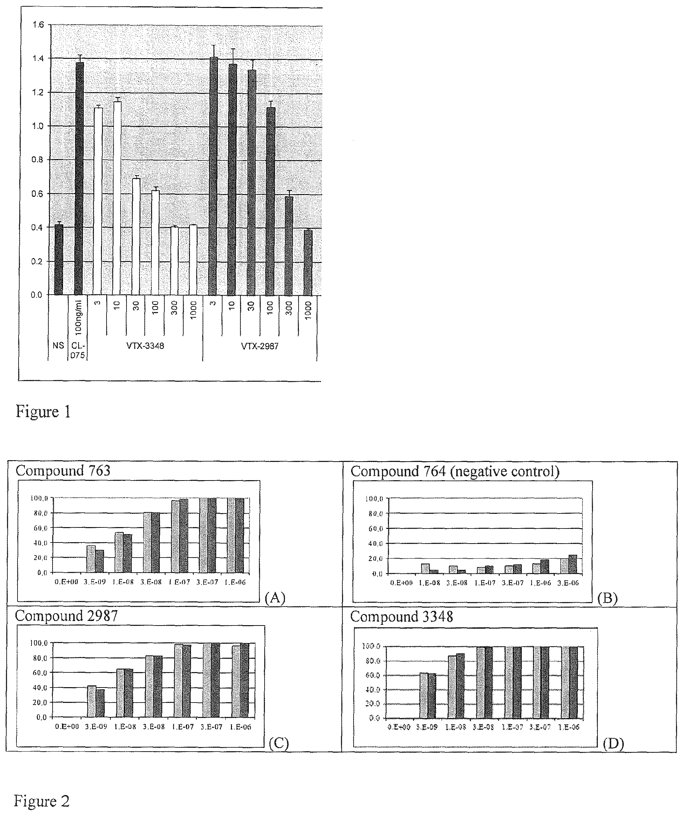

The antagonist activity of the compounds of this invention was further demonstrated using human peripheral blood mononuclear cells (PBMCs). PBMCs contain a mixture of cells including monocytes and myeloid dendritic cells (mDCs) that express TLR8. When stimulated with the small molecule TLR8 agonists, PBMCs produce increased levels of IL-8. The ability of TLR8 antagonists to inhibit TLR8 production in human PBMCs was evaluated. Dose depending inhibition was observed when cells with stimulated with CL075, a structurally distinct thiazoquinoline TLR8 agonist. Figure 1 shows dose-dependent inhibition of IL-8 production in human PBMC stimulated with CL075. Data shown in Figure 1 are a representative experiment from one donor evaluated in duplicate culture wells. Increasing concentrations (from 3 to 1000 nM) of Compounds 3348, 2987, 3261, 3387, and 3448 (labeled as VTX-3348, VTX-2987, VTX-3261, VTX-3387, VTX-3448 in Figure 1) were added to human PBMCs (50,000 cells/ well in RPMI) and incubated for 2 hours in a 37°C humidified CO₂ incubator. CL075 (Invivogen) was added to a final concentration of 100 ng/ML (400 nM) and cell were incubated overnight. At the end of the incubation, cells were centrifuged and cell culture supernatants were analyzed for IL-8 by ELISA (eBiosciene kit) per the manufacturer's instructions. The absorbance (OD 450 nM) representative of IL-8 levels is shown on the y-axis of Figure 1. In the absence of any TLR8 agonists or antagonists (NS) the OD was 0.417 and addition of CL075 increased OD to 1.3777 (first two bars at the left of Figure 1). As demonstrated in Figure 1, in the presence of increasing concentrations of TLR8 antagonists, IL-8 levels were reduced in a dose dependent fashion.

The experiment shown in Figure 1 was repeated in multiple donors and with additional TLR8 antagonist molecules (see Figure 2). Cells were stimulated with CL075 (100 ng/mL) and inhibition of IL-8 production was measured as described in Figure 1. Percent inhibition is shown on the y-axis and concentrations of TLR8 antagonists (3-1000 nM) are shown on the x-axis in Figure 2. Compound 764 has the structure:

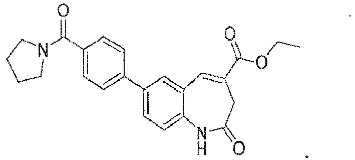

The foregoing description is considered as illustrative only of the principles of the invention. Further, since numerous modifications and changes will be readily apparent to those skilled in the art, it is not desired to limit the invention to the exact construction and process shown as described above. Accordingly, all suitable modifications and equivalents may be resorted to falling within the scope of the invention as defined by the claims that follow.

The words "comprise," "comprising," "include," "including," and "includes" when used in this specification and in the following claims are intended to specify the presence of stated features, integers, components, or steps, but they do not preclude the presence or addition of one or more other features, integers, components, steps, or groups thereof.

### INCORPORATION BY REFERENCE

The entire disclosure of each of the patent documents and scientific articles referred to herein is incorporated by reference for all purposes.

### EQUIVALENTS

The invention can be embodied in other specific forms without departing from the spirit or essential characteristics thereof. The foregoing embodiments are therefore to be considered in all respects illustrative rather than limiting on the invention described herein. Scope of the invention is thus indicated by the appended claims rather than by the foregoing description, and all changes that come within the meaning and range of equivalency of the claims are intended to be embraced therein.

Preferred embodiments of the invention include:
A compound having the formula I or a pharmaceutically acceptable salt thereof: 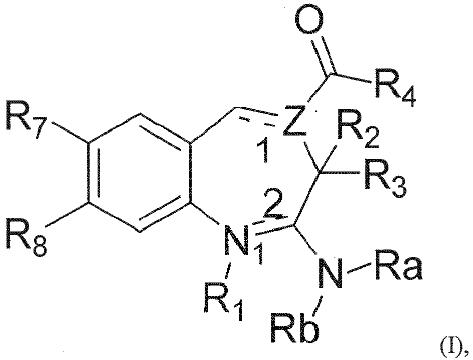 wherein
   -̅-̅-̅-̅-̅-̅ is a double bond or a single bond;
   R₂ and R₃ are independently selected from H and lower alkyl, or R₂ and R₃ are connected to form a saturated carbocycle having from 3 to 7 ring members; one of R₇ and R₈ is 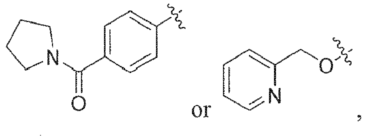 and the other is hydrogen;
   R₄ is -NR_{c}R_{d} or -OR₁₀;
   R_{c} and R_{d} are lower alkyl optionally substituted with one or more -OH;
   R₁₀ is alkyl optionally substituted with one or more -OH;
   Z is C and -̅-̅-̅-̅-̅-̅ is a double bond, or Z is N and -̅-̅-̅-̅-̅-̅ is a single bond;
   Rₐ and R_{b} are independently selected from H, alkyl, alkenyl, alkynyl, and Rₑ, wherein the alkyl is optionally substituted with one or more -OR₁₀, or Rₑ;
   Rₑ is selected from -NH₂, -NH(alkyl), and -N(alkyl)₂;
   2 2 R₁ is absent when -̅-̅-̅-̅-̅-̅ is a double bond, or when -̅-̅-̅-̅-̅-̅ is a single bond, N₁-R₁ and one of Rₐ or R_{b} are connected to form a saturated, partially unsaturated, or unsaturated heterocycle having 5-7 ring members and the other of Rₐ or R_{b} may be hydrogen or absent as necessary to accommodate ring unsaturation; and
   at least one of the following A-D applies:

A) R₇ is not hydrogen;
B) R₈ is not hydrogen and at least one of Rₐ and R_{b} is not hydrogen;
C) Z is N; or
D) N₁-R₁ and one of Rₐ or R_{b} are connected to form a saturated, partially unsaturated, or unsaturated heterocycle having 5-7 ring members.

The compound as defined above, wherein R₇ is

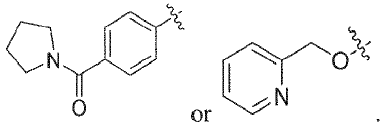

The compound as defined above, wherein at least one of Rₐ and R_{b} is not hydrogen.

The compound as defined above, wherein one of Rₐ and R_{b} is alkyl and the other of Rₐ and R_{b} is hydrogen.

The compound as defined above, wherein one of Rₐ and R_{b} is alkyl substituted with Rₑ.

The compound as defined above, wherein both Rₐ and R_{b} are alkyl.

The compound as defined above, wherein one of Rₐ and R_{b} is Rₑ, and the other of Rₐ and R_{b} is hydrogen.

The compound as defined above, wherein R₈ is not hydrogen.

The compound as defined above, wherein N₁ and one of Rₐ or R_{b} are connected to form a saturated, partially unsaturated, or unsaturated heterocycle having 5-7 ring members and the other of Rₐ or R_{b} is hydrogen, or absent as necessary to accommodate ring unsaturation.

The compound as defined above, wherein N₁ and one of Rₐ or R_{b} are connected to form a saturated, partially unsaturated, or unsaturated heterocycle having 5 ring members.

The compound as defined above, wherein N₁ and one of Rₐ or R_{b} are connected to form

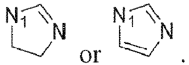

The compound as defined above, wherein at least one of R₂ and R₃ is not hydrogen.

The compound as defined above, wherein R₂ and R₃ are connected to form a saturated carbocycle.

The compound as defined above, wherein R₂ and R₃ are connected to form cyclopropyl.

The compound as defined above, wherein Z is N.

A compound having the formula II or a pharmaceutically acceptable salt thereof: 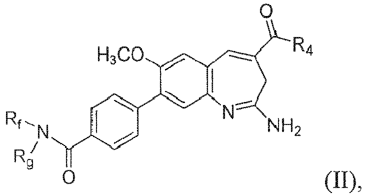 wherein
R₄ is -NR_{c}R_{d} or -OR₁₀;
R_{c} and R_{d} are lower alkyl optionally substituted with one or more -OH;
R₁₀ is alkyl optionally substituted with one or more -OH;
R_{f} and R_{g} are lower alkyl, or R_{f} and R_{g} together with the nitrogen atom to which they are attached form a saturated heterocyclic ring having 4-6 ring members.

The compound as defined above, wherein R_{f} and R_{g} together with the nitrogen atom to which they are attached form a saturated heterocyclic ring.

The compound as defined above, wherein R_{f} and R_{g} together with the nitrogen atom to which they are attached form pyrrolidine.

The compound as defined above, wherein R₄ is -OR₁₀ and R₁₀ is alkyl.

The compound as defined above, wherein R₄ is -O-ethyl.

The compound as defined above, wherein R₄ is -NR_{c}R_{d} and R_{c} and R_{d} are both alkyl.

The compound as defined above, wherein R₄ is -N(propyl)₂.

The compound as defined above, wherein at least one of R_{c} or R_{d} is alkyl substituted with one -OH.

The compound as defined above, wherein at least one of R_{c} and R_{d} is 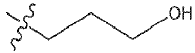 and the remaining R_{c} or R_{d} is propyl.

### A compound selected from

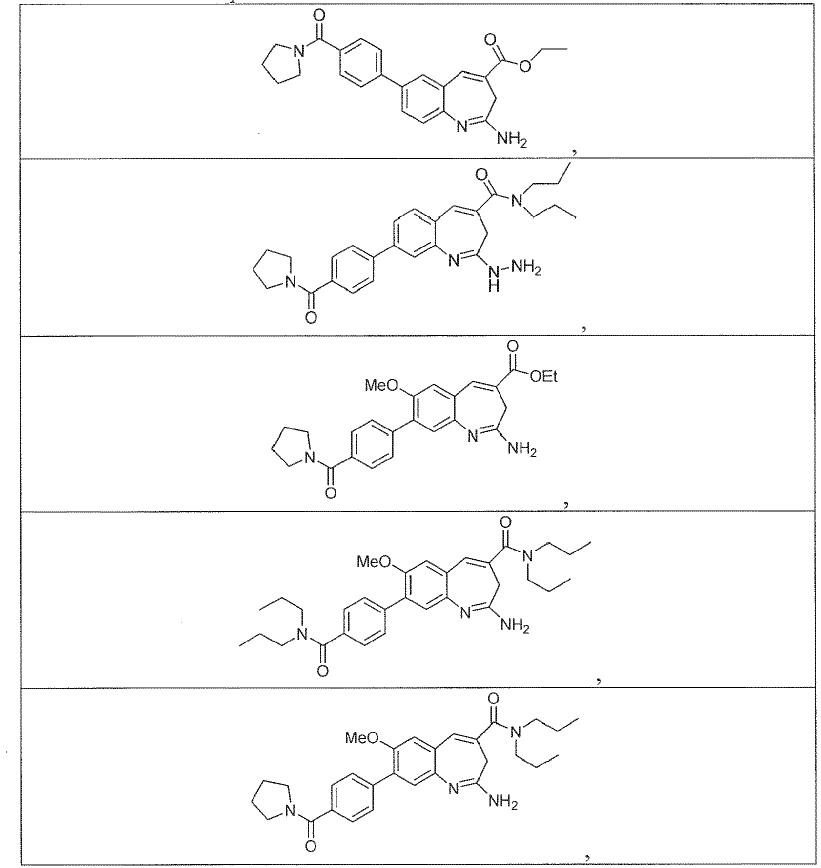

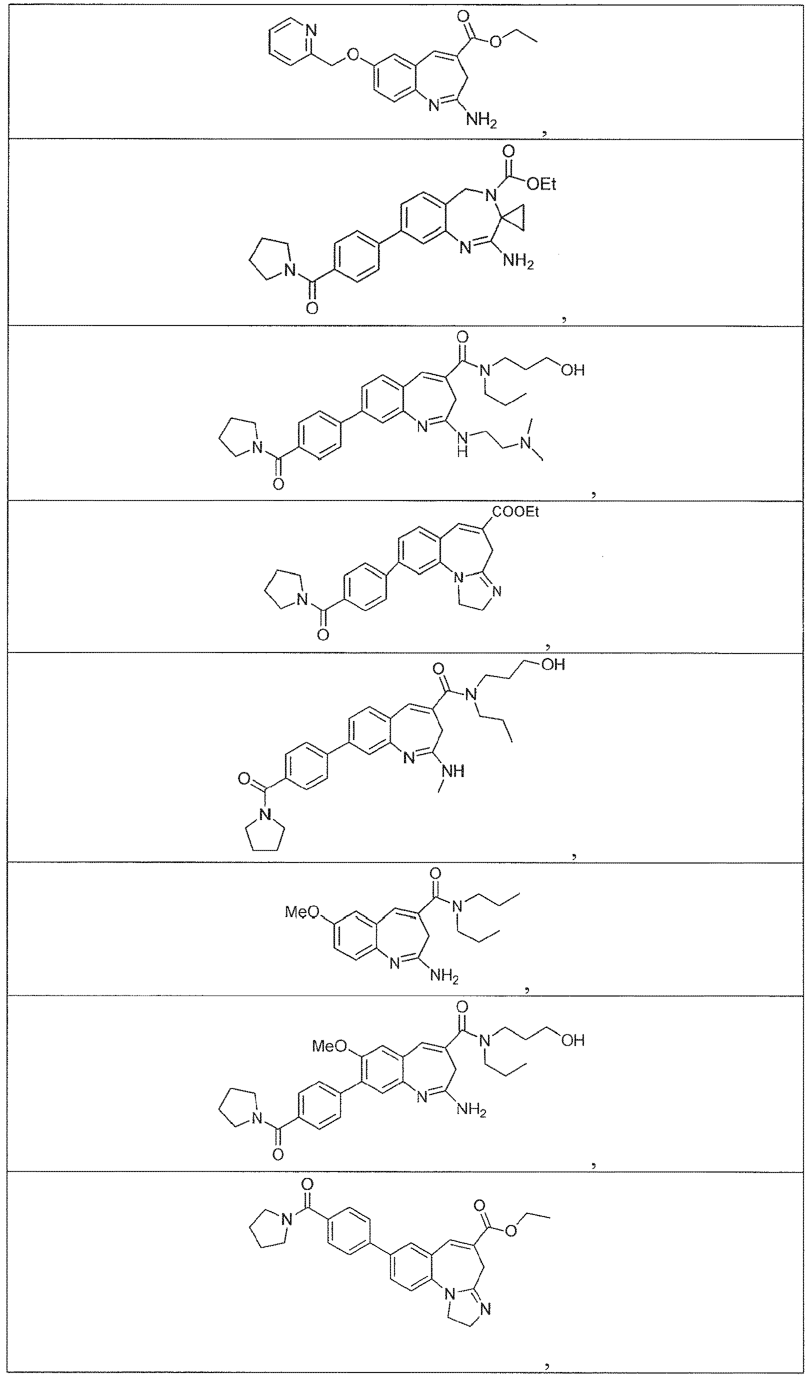

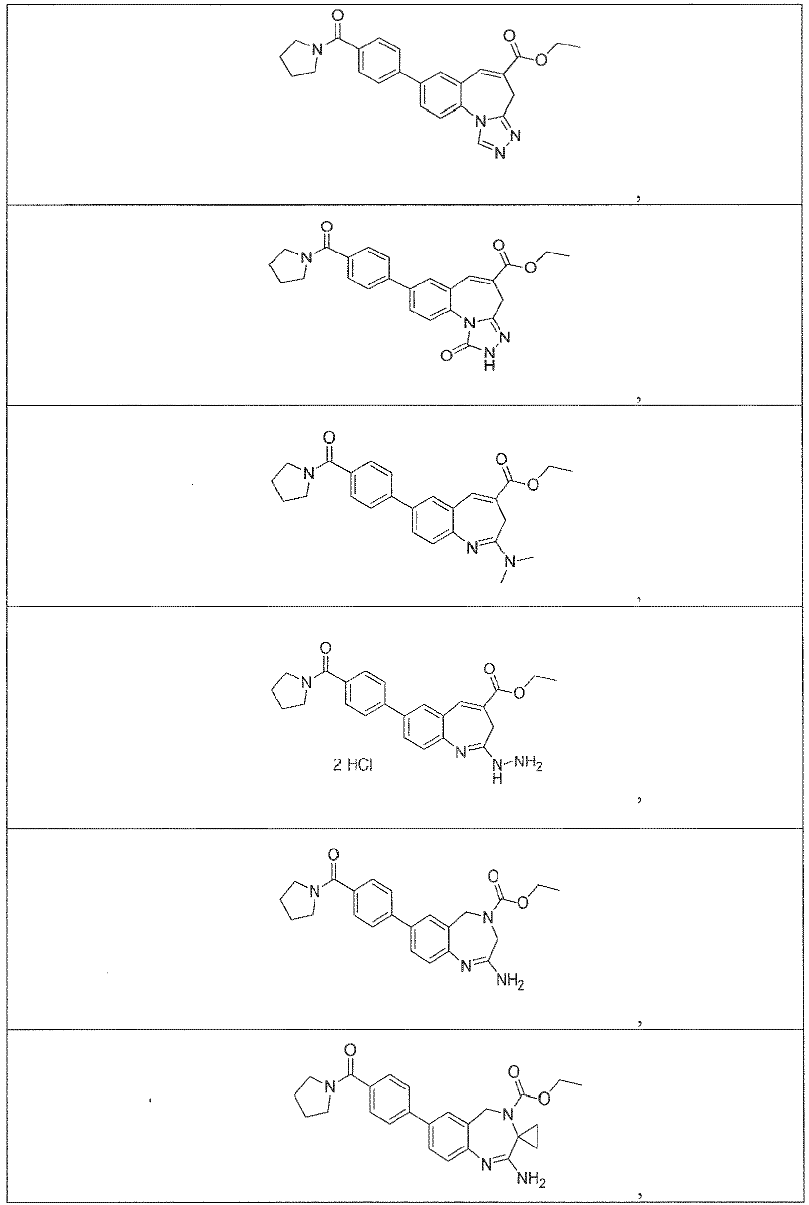

and pharmaceutically acceptable salts thereof.

A compound having the structure: 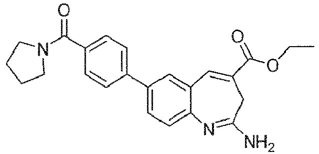 or a pharmaceutically acceptable salt thereof.

A pharmaceutical composition comprising a compound as defined above or a pharmaceutically acceptable salt thereof and a pharmaceutically acceptable diluent or carrier.

A compound as defined above or a pharmaceutically acceptable salt thereof for use in the treatment of a TLR7- and/or TLR8-mediated condition.

A compound as defined above or a pharmaceutically acceptable salt thereof for use in the treatment of an autoimmune condition.

## Claims

1. A compound having the formula II or a pharmaceutically acceptable salt thereof: 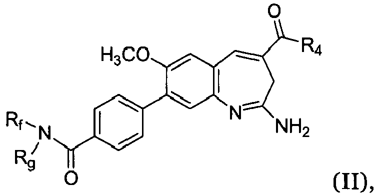 wherein
R₄ is -NR_{c}R_{d} or -OR₁₀;
R_{c} and R_{d} are lower alkyl optionally substituted with one or more -OH;
R₁₀ is alkyl optionally substituted with one or more -OH;
R_{f} and R_{g} are lower alkyl, or R_{f} and R_{g} together with the nitrogen atom to which they are attached form a saturated heterocyclic ring having 4-6 ring members.

2. The compound of claim 1, wherein R_{f} and R_{g} together with the nitrogen atom to which they are attached form a saturated heterocyclic ring, preferably R_{f} and R_{g} together with the nitrogen atom to which they are attached form pyrrolidine.

3. The compound of claim 1, wherein R₄ is -OR₁₀ and R₁₀ is alkyl, preferably R₄ is -O-ethyl; or R₄ is -NR_{c}R_{d} and R_{c} and R_{d} are both alkyl, preferably R₄ is -N(propyl)₂.

4. The compound of claim 1, wherein at least one of R_{c} or R_{d} is alkyl substituted with one - OH, preferably at least one of R_{c} and R_{d} is 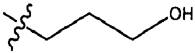 and the remaining R_{c} or R_{d} is propyl.

5. A compound selected from 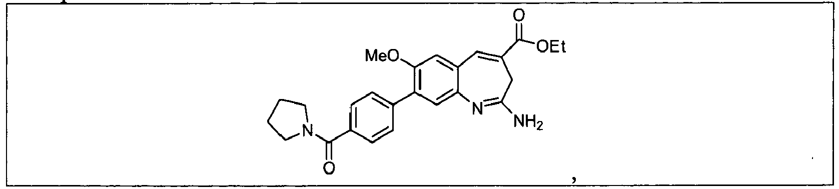 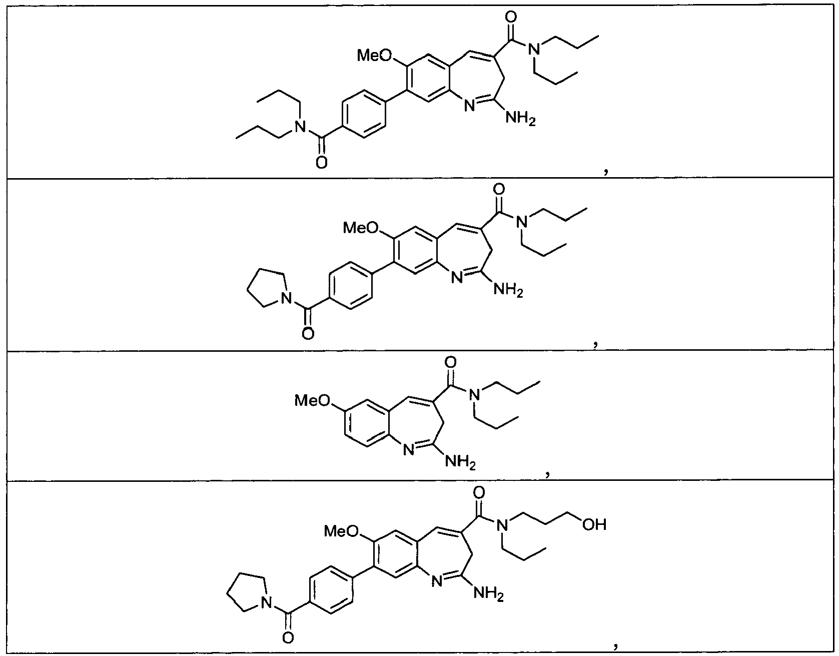 or a pharmaceutically acceptable salt thereof.

6. A pharmaceutical composition comprising a compound of any one of claims 1-5 or a pharmaceutically acceptable salt thereof and a pharmaceutically acceptable diluent or carrier.

7. A compound of any one of claims 1-6 or a pharmaceutically acceptable salt thereof for use in the treatment of a condition selected from cancer, an immune complex-associated disease, autoimmune disease, infectious disease, inflammatory disorder, immunodeficiency, graft rejection, graft-versus-host disease, allergy, cardiovascular disease, fibrotic disease, asthma, and sepsis.
